(19) Europäisches Patentamt
European Patent Office
Office européen des brevets

(11) **EP 2 638 850 B1**

(12) **EUROPEAN PATENT SPECIFICATION**

(45) Date of publication and mention
of the grant of the patent:
**10.08.2016 Bulletin 2016/32**

(51) Int Cl.:
**A61B 5/00** (2006.01)   **A61B 5/1455** (2006.01)

(21) Application number: **13000838.6**

(22) Date of filing: **19.02.2013**

(54) **Subject information obtaining device, subject information obtaining method, and program**

Vorrichtung zum Erhalt von Subjektinformationen, Verfahren zum Erhalt von Subjektinformationen und Programm

Dispositif d'obtention d'informations d'un sujet, procédé d'obtention d'informations d'un sujet et programme

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2012 JP 2012056015**

(43) Date of publication of application:
**18.09.2013 Bulletin 2013/38**

(73) Proprietor: **Canon Kabushiki Kaisha Tokyo (JP)**

(72) Inventor: **Oishi, Takuji Ohta-ku, Tokyo (JP)**

(74) Representative: **WESER & Kollegen Radeckestraße 43 81245 München (DE)**

(56) References cited:
**US-A1- 2009 227 870     US-A1- 2010 049 049 US-A1- 2010 094 561**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

One disclosed aspect of the embodiments relates to a subject information obtaining device, a subject information obtaining method, and a program, which obtain subject information by detecting photoacoustic waves generated by light being irradiated.

Description of the Related Art

With the medical field, in recent years, photoacoustic imaging (PAI) whereby living body function information is obtained using light and ultrasonic waves has been proposed as one of devices capable of imaging the interior of a living body noninvasively, and its development has advanced.

The photoacoustic imaging mentioned here is technology to irradiate pulsed light generated from a light source upon a subject, and image internal tissue serving as a generation source of photoacoustic waves, using photoacoustic advantages that photoacoustic waves (typically, ultrasonic waves) are generated by absorption of light spread and diffused within the subject. Specifically, signals obtained by detecting change resulting from time of received photoacoustic waves at multiple locations are mathematically analyzed, i.e., restructured, and information relating to an optical characteristic value within a subject is visualized three-dimensionally.

In the event of employing near-infrared light as pulsed light, near-infrared light has a property to readily penetrate water making up a majority portion of a living body and to readily be absorbed by hemoglobin in blood, which enables a blood vessel image to be imaged. Further, oxygen saturation in blood, which is function information, may be measured by comparing blood vessel images obtained from pulsed light beams with different wavelengths. It has been thought that blood around a malignant tumor is lower in oxygen saturation than blood around a benign tumor, and accordingly, there is anticipation that malignant/benign judgment of a tumor may be performed by knowing oxygen saturation.

Japanese Patent Laid-Open No. 2010-35806 has disclosed that a concentration distribution of a substance making up a living body is imaged by the photoacoustic imaging.

However, as disclosed in Japanese Patent Laid-Open No. 2010-35806, with the photoacoustic imaging, at the time of imaging photoacoustic waves, artifacts emerging in a position where there is actually no optical absorber hinder observation of optical absorbers.

For example, in the event that a subject is held at a holding plate of which the acoustic impedance differs from a subject, photoacoustic waves generated within the subject are reflected multiply within the holding plate. At the time of detecting photoacoustic waves thus reflected multiply for imaging, artifacts due to multiple reflections emerge. Such artifacts hinder an optical absorber image actually existing from being distinguished.

Therefore, it has been found to be desirable to provide a subject information obtaining device and a subject information obtaining method whereby information with an optical absorber image or artifacts being enhanced may be obtained.

US 2010/0049049 A1 discloses a biological information imaging apparatus comprising: a measurement unit that measures an acoustic wave emitted from a tissue in a living body at a time when the tissue absorbs light irradiated onto the living body; a storage unit that stores first information obtained in a first measurement by the measurement unit; and an information processing unit that generates an image of information of the tissue in the living body by using second information obtained in a second measurement by the measurement unit and the first information stored in the storage unit.

SUMMARY OF THE INVENTION

The present invention in its first aspect provides a subject information obtaining device as specified in claims 1 to 12.

The present invention in its second aspect provides a subject information obtaining method as specified in claim 13.

The present invention in its third aspect provides program as specified in claim 14.

Further features of the present invention will become apparent from the following description of exemplary embodiments with reference to the attached drawings.

BRIEF DESCRIPTION OF THE DRAWINGS

Figs. 1A to 1E are diagrams for describing principles of the present disclosure.

Fig. 2 is a diagram illustrating a configuration of a subject information obtaining device according to a first embodiment.

Fig. 3 is a diagram illustrating a flowchart for a subject information obtaining method according to the first embodiment.

Figs. 4A and 4B are diagrams illustrating an example of a first measurement state and a second measurement state according to the first embodiment.

Figs. 5A and 5B are diagrams illustrating another example of the first measurement state and second measurement state according to the first embodiment.

Figs. 6A and 6B are diagrams illustrating another example of the first measurement state and second measurement state according to the first embodiment.

Figs. 7A and 7B are diagrams illustrating another example of the first measurement state and second measurement state according to the first embodiment.

Figs. 8A and 8B are diagrams illustrating another example of the first measurement state and second measurement state according to the first embodiment.

Figs. 9A to 9C are diagrams illustrating an example of a method to obtain a similarity distribution, according to the first embodiment.

Fig. 10 is a diagram illustrating an example of a display device where a similarity distribution is displayed, according to the first embodiment.

Fig. 11 is a diagram illustrating a flowchart for a subject information obtaining method according to a second embodiment.

Fig. 12 is a diagram illustrating an example of a display device where a similarity distribution and a light intensity distribution are displayed, according to the second embodiment.

Fig. 13 is a diagram illustrating a flowchart for a subject information obtaining method according to a third embodiment.

Figs. 14A and 14B are diagrams illustrating an example of a display device where a similarity distribution and confidence regions are displayed, according to the third embodiment.

DESCRIPTION OF THE EMBODIMENTS

Artifacts are observed in the same way if measurement is performed in the same way since artifacts are high in reproducibility. Therefore, artifacts emerge in the same way with measurement, and accordingly, an optical absorber image and artifacts are not readily distinguished.

With the present disclosure, with the photoacoustic imaging, a similarity distribution is obtained illustrating height of the similarity of each optical characteristic value distribution obtained by multiple measurements with a different measurement state. The similarity distributions thus obtained becomes distributions where an optical absorber image or artifacts are enhanced.

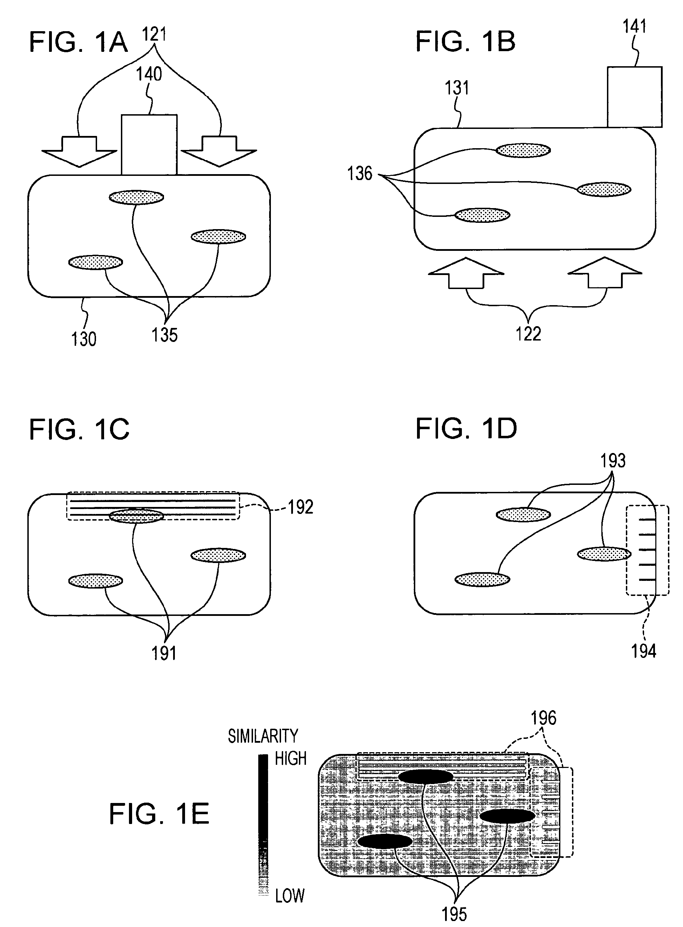

First, principles of the present disclosure will be described with reference to Figs. 1A to 1E. Figs. 1A to 1E illustrate an image of a position relation (measurement state) between irradiation light and an acoustic wave detector and a subject, and an initial sound pressure distribution obtained at the time of this position relation. Here, subjects 130 and 131 include optical absorbers 135 and 136 in the same positions, respectively.

With a measurement state illustrated in Fig. 1A, light 121 in a first measurement state is irradiated on a surface of the subject 130 acoustically connected to a detection surface of an acoustic wave detector 140.

Also, with a measurement state illustrated in Fig. 1B, light 122 in a second measurement state is irradiated on a surface of the subject 131 facing an acoustic wave detector 141. Also, in Fig. 1B, a portion of the acoustic wave detector 141 is not acoustically connected to the subject 131.

Further, an initial sound pressure distribution obtained at the time of the measurement state in Fig. 1A is illustrated in Fig. 1C. Also, an initial sound pressure distribution obtained at the time of the measurement state in Fig. 1B is illustrated in Fig. 1D.

It is understandable that appearing positions of artifacts 192 and 194 differ depending on measurement states when comparing Figs. 1C and 1D. On the other hand, it is understandable that appearing positions of optical absorber images 191 and 193 are the same even when changing the measurement state. This is because a process for occurrence of artifacts differs depending on measurement states. Hereinafter, the process for occurrence of artifacts in each of the measurement states will be described.

With the measurement state in Fig. 1A, the light 121 in the first measurement state also partially generates wraparound photoacoustic waves on the surface of the acoustic wave detector 140. The photoacoustic waves generated on the surface of the acoustic wave detector 140 then appear as the artifacts 192 illustrated in Fig. 1C due to multiple reflections or the like in an acoustic matching layer of the acoustic wave detector 140 or the like.

Also, with the measurement state in Fig. 1B, of the acoustic wave detector 141, the light 122 in the second measurement state wrapped around the subject 131 directly inputs to a portion which is not acoustically connected to the subject 131 to generate photoacoustic waves on the surface of the acoustic wave detector 141. The photoacoustic waves then appear as the artifacts 194 illustrated in Fig. 1D due to multiple reflections or the like in the acoustic matching layer of the acoustic wave detector 141.

Next, an initial sound pressure distribution illustrated in Fig. 1C where the appearance positions of the artifacts differ, and a similarity distribution illustrated in Fig. 1E illustrating height of similarity for the initial sound pressure distribution illustrated in Fig. 1D are obtained. However, with the initial sound pressure distributions illustrated in Figs. 1C and 1D, there is an image where random noise has been imaged in the background other than optical absorber images and the regions of the artifacts. Therefore, similarity of the background illustrated in Fig. 1E is smaller than the regions of optical absorber images 195, and greater than the regions of artifacts 196.

The optical absorber images appear in the same positions in the initial sound pressure distribution illustrated in Fig. 1C, and in the initial sound pressure distribution illustrated in Fig. 1D, and accordingly, as illustrated in Fig. 1E, similarity corresponding to the optical absorber images 195 is illustrated high.

On the other hand, the artifacts emerge, both in the initial sound pressure distribution illustrated in Fig. 1C and in the initial sound pressure distribution illustrated in Fig. 1D, different positions, and accordingly, similarity corresponding to the artifacts 196 is illustrated low in Fig. 1E.

As described above, with the similarity distribution of each optical characteristic value distribution obtained in the multiple measurement states, the similarity of the optical absorber images is illustrated high, and the similarity of the artifacts is illustrated low. Therefore, optical absorber images and artifacts may be distinguished by displaying a similarity distribution.

Note that, with the present disclosure, the optical characteristic value distributions include an initial sound pressure distribution obtained by performing reconstitution processing on a detection signal, and an optical energy density distribution, or a distribution relating to an optical absorption coefficient obtained by performing light intensity correction thereon. Also, the optical characteristic value distributions include data to be displayed on a display unit, obtained by performing luminance value conversion on those distributions.

Also, the similarity distributions also include data to be displayed on the display unit, obtained by performing luminance value conversion on a similarity distribution.

Hereinafter, embodiments of the present disclosure will be described.


First Embodiment

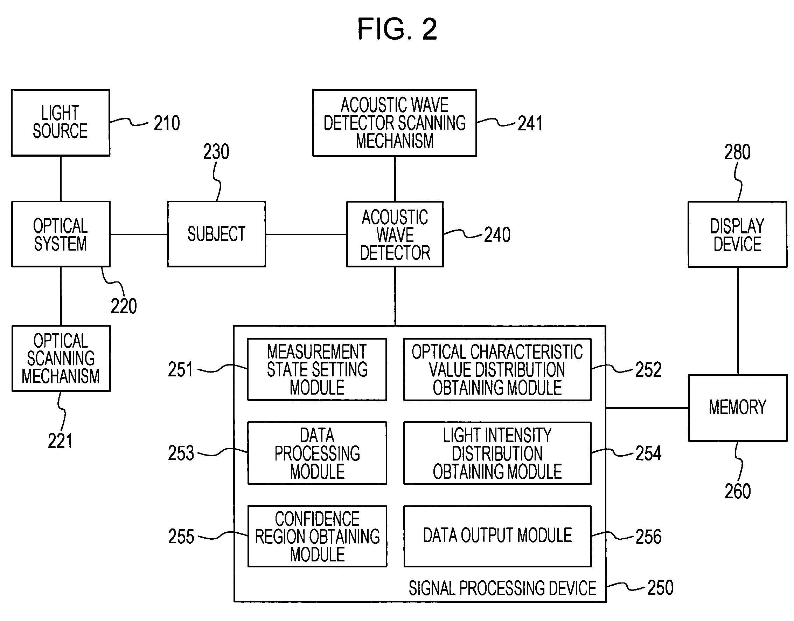

Fig. 2 is a block diagram illustrating a configuration of a subject information obtaining device according to the present embodiment, and is configured of a light source 210, an optical system 220, an optical scanning mechanism 221, a subject 230, an acoustic wave detector 240, an acoustic wave detector scanning mechanism 241, a signal processing device 250, memory 260, and a display device 280. The signal processing device 250 according to the present embodiment includes a measurement state setting module 251 serving as a measurement state setting unit, an optical characteristic value distribution obtaining module 252 serving as an optical characteristic value distribution obtaining unit, a data processing module 253 serving as a data processing unit, a light intensity distribution obtaining module 254 serving as a light intensity distribution obtaining unit, a confidence region obtaining module 255 serving as a confidence region obtaining unit, and a data output module 256.

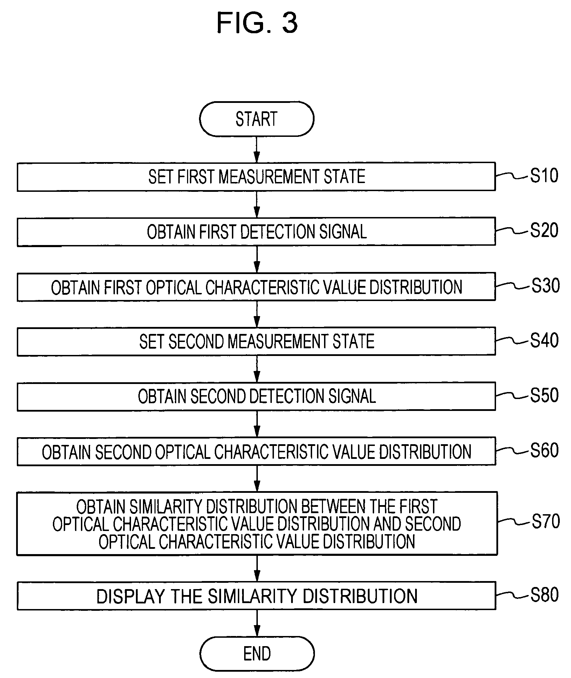

Fig. 3 is a diagram illustrating a flow of a subject information obtaining method using the subject information obtaining device according to the present embodiment illustrated in Fig. 2.

First, the measurement state setting module 251 controls the light source 210, optical system 220, and acoustic wave detector 240 to set these in the first measurement state, and light in the first measurement state is irradiated on the subject 230 (S10). The acoustic wave detector 240 then detects first photoacoustic waves generated at the subject 230 by the light in the first measurement state to obtain a first detection signal (S20).

Next, the optical characteristic value distribution obtaining module 252 serving as a first optical characteristic value distribution obtaining unit performs reconstitution processing using this first detection signal, thereby obtaining a first initial sound pressure distribution serving as a first optical characteristic value distribution, and saving this in the memory 260 (S30).

Next, the measurement state setting module 251 sets the light source 210, optical system 220, and acoustic wave detector 240 in the second measurement state, and light in the second measurement state is irradiated on the subject 230 (S40). The acoustic wave detector 240 then detects second photoacoustic waves generated at the subject 230 using the light in the second measurement state to obtain a second detection signal (S50).

Next, the optical characteristic value distribution obtaining module 252 serving as a second optical characteristic value distribution obtaining unit performs reconstitution processing using this second detection signal, thereby obtaining a second initial sound pressure distribution serving as a second optical characteristic value distribution, and saving this in the memory 260 (S60).

Next, the data processing module 253 obtains a similarity distribution between the first optical characteristic value distribution and second optical characteristic value distribution to save this in the memory 260 (S70).

The data output module 256 then outputs the similarity distribution saved in the memory 260 to the display device 280, and displays the similarity distribution on the display device 280 (S80).

Note that the optical characteristic value distribution obtaining module 252 may obtain an initial sound pressure distribution by performing reconstitution processing using a heretofore known reconstituting method such as a universal back-projection method to overlap signals subjected to differential processing, for example, as described in (Minghua Xu and Lihong V. Wang, (2005), "Universal back-projection algorithm for photoacoustic computed tomography", PHYSICAL REVIEW E 71, 016706), or the like.

Also, a program including the above-mentioned processes may be executed by the signal processing device 250 serving as a computer.

S10, S40: Processes to Set Measurement State

Next, setting for a measurement state illustrated in S10 and S40 in Fig. 3 will be described in detail.

Hereinafter, description will be made regarding an example wherein the measurement state setting module 251 sets a measurement state. The measurement states in the present disclosure are conception including an irradiated state (irradiated position, irradiated angle, and irradiated intensity) of irradiation light and the detection position of an acoustic wave detector.

First, description will be made regarding an example wherein the irradiated state of irradiation light serving as a measurement state is changed.

1. Example for Irradiating Light in Different Positions of Subject Surface

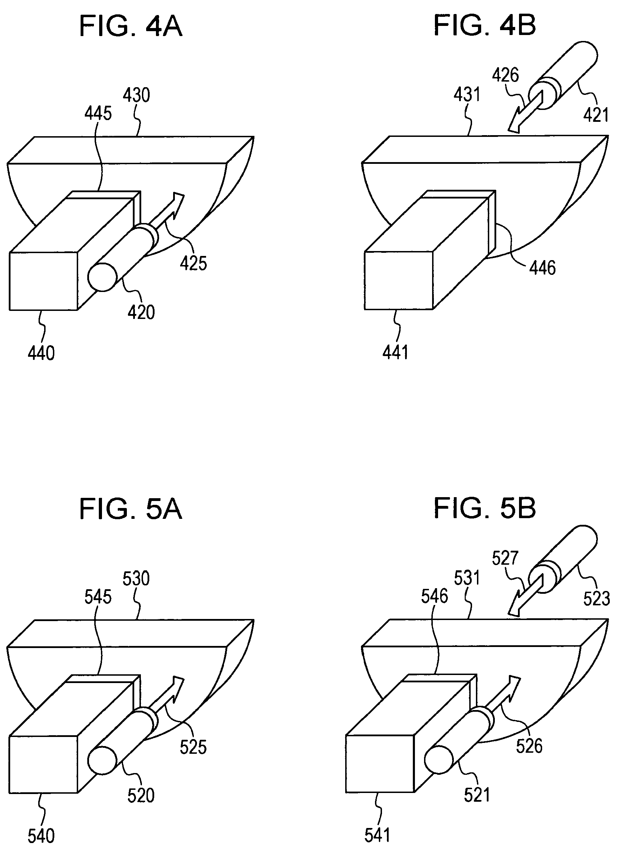

First, description will be made regarding an example of a measurement state in which light in a first measurement state and light in a second measurement state are irradiated on different positions of a subject surface, with reference to Figs. 4A, 4B, 5A, and 5B.

With a first measurement state illustrated in Fig. 4A, light 425 in the first measurement state is irradiated on a surface of a subject 430 acoustically connected to a detection surface 445 of an acoustic wave detector 440 from an optical system 420. Next, with a second measurement state illustrated in Fig. 4B, light 426 in the second measurement state is irradiated on a surface facing a surface of a subject 431 acoustically connected to a detection surface 446 of an acoustic wave detector 441 from an optical system 421.

In this manner, with each of the measurement states, in the event that light is irradiated on different positions of a subject surface, and each of the measurement results is taken as an optical characteristic value distribution, processes for occurrence of artifacts differ, and accordingly, artifacts emerge in different positions. On the other hand, as described above, with an optical characteristic value distribution obtained in each measurement state, optical absorber images emerge in the same position.

Therefore, with a similarity distribution of each optical characteristic value distribution obtained by irradiating light on different positions of the subject surface, similarity of optical absorber images is illustrated high, and similarity of artifacts is illustrated low.

Also, even with the measurement states illustrated in Figs. 5A and 5B, the present disclosure may be applied. The first measurement state illustrated in Fig. 5A is the same as the measurement state illustrated in Fig. 4A. On the other hand, with the second measurement state illustrated in Fig. 5B, light 525 in the same measurement state as the measurement state illustrated in Fig. 4A, and light 526 in the same measurement state as the measurement state illustrated in Fig. 4B are irradiated on the surfaces of a subject 531.

In this manner, even in the event that the light in the second measurement state includes not only the light in the first measurement state but also the light irradiated on a position of the subject surface different from the light in the first measurement state, positions and intensities of artifacts occurring differ.

Therefore, in this case as well, with a similarity distribution of the optical characteristic value distribution in each measurement state, similarity of optical absorber images is illustrated high, and similarity of artifacts is illustrated low.

Note that irradiated position for the subject surface may be changed by changing a beam profile of light in the subject surface in each measurement state.

Also, the greater a position to be irradiated of the subject surface is changed depending on measurement states, the greater an occurrence position of artifacts is also changed, and accordingly, it is desirable to greatly change an irradiated position depending on measurement states.

2. Example for Irradiating Light with Different Angle Made Up of Subject Surface and Irradiation Direction

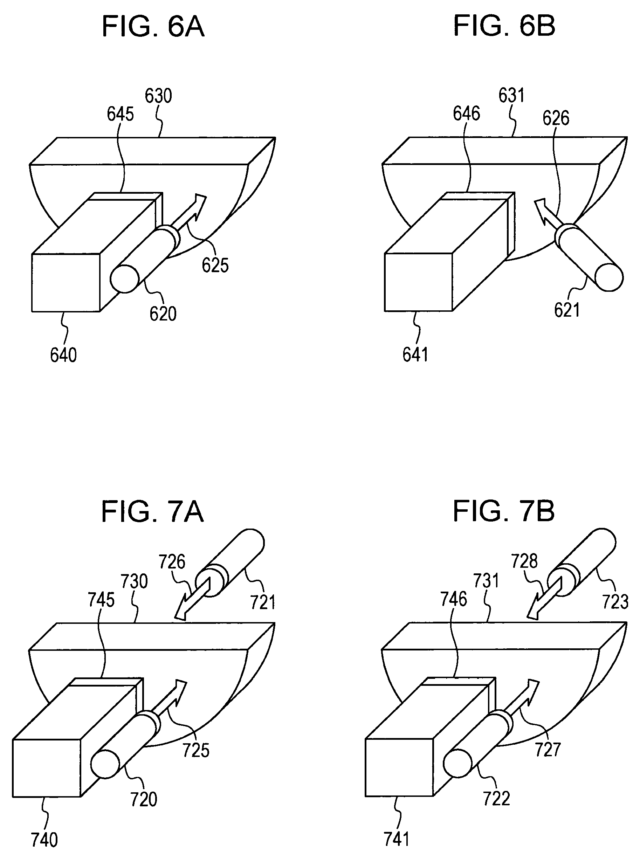

Next, description will be made regarding an example of a measurement state with a different irradiation angle which is an angle made up of a subject surface and the irradiation direction of light, with reference to Figs. 6A and 6B.

A first measurement state illustrated in Fig. 6A is the same as the measurement state illustrated in Fig. 4A. On the other hand, with a second measurement state illustrated in Fig. 6B, light 626 in the second measurement state is irradiated on the same position as an irradiation position of light 625 in the first measurement state illustrated in Fig. 6A with a irradiation angle different from the light 625 in the first measurement state from an optical system 621.

In this case as well, when comparing artifacts in an optical characteristic value distribution obtained in the measurement state illustrated in Fig. 6A, and an optical characteristic value distribution obtained in the measurement state illustrated in Fig. 6A, positions where artifacts emerge differ.

Accordingly, with a similarity distribution of each optical characteristic value distribution obtained from multiple measurement states with a different angle made up of the subject surface and the irradiation direction, similarity of optical absorber images is illustrated high, and similarity of artifacts is illustrated low.

Note that the greater the irradiation angle is changed depending on measurement states, the greater an occurrence position of artifacts is also changed, and accordingly, it is desirable to greatly change the irradiation angle depending on measurement states.

3. Example for Irradiating Light with Different Irradiation Intensity

Next, description will be made regarding an example of a measurement state with different irradiation intensity of light in each measurement state, with reference to Figs. 7A and 7B.

With the measurement states illustrated in Figs. 7A and 7B, an optical system includes multiple light irradiating units, and multiple light beams are irradiated on different positions of a subject surface from each of the light irradiating units.

Here, light 725 in a first measurement state illustrated in Fig. 7A, and light 727 in a second measurement state illustrated in Fig. 7B are irradiated on the same position, and light 726 in a first measurement state illustrated in Fig. 7A, and light 728 in a second measurement state illustrated in Fig. 7B are irradiated on the same position.

With the measurement state illustrated in Fig. 7A, the light 725 with weak intensity is irradiated on a surface of a subject 730 acoustically connected to an acoustic wave detector 740, and the light 726 with strong intensity is irradiated on a surface of the subject 730 facing that surface.

On the other hand, with the measurement state illustrated in Fig. 7B, the light 727 with strong intensity is irradiated on a surface of a subject 731 acoustically connected to an acoustic wave detector 741, and the light 728 with weak intensity is irradiated on a surface of the subject 731 facing that surface.

When light is irradiated from the multiple light irradiating units, photoacoustic waves corresponding to each irradiation light beam from each light irradiating unit occur. Artifacts corresponding to the intensity of each irradiation light then emerge, and an optical characteristic value distribution to which the artifacts thereof are added is obtained. Accordingly, signal intensities of artifacts differ depending on measurement states.

Accordingly, with a similarity distribution of each optical characteristic value distribution obtained from multiple measurement states with different intensity of irradiation light, similarity of optical absorber images is illustrated high, and similarity of artifacts is illustrated low.

As a method to change irradiation intensity of light in each measurement state, there are conceived a method for providing a filter to attenuate light to an optical system, a method to adjust output of a light source corresponding to light in each measurement state, and so forth. Additionally, any method may be employed as long as the method enables to change irradiation intensity of light in each measurement state.

As described above, with the method to change the measurement state by changing multiple irradiation intensities, as compared to a method to change the irradiation position or irradiation angle, driving of the optical system may be reduced. In particular, in the event of adjusting output of the light source, driving of the optical system may considerably be reduced. Therefore, mechanical movement of the device may be reduced, and automation of measurement may simply be realized.

Note that, in the event that light is irradiated from the multiple light irradiating units, it is desirable to change irradiation intensity in each measurement state with the optical system fixed. Precision of the irradiation position may be improved by fixing the optical system.

With the present disclosure, that the irradiation intensity of light in the first measurement state and the irradiation intensity of light in the second measurement differ indicates that the irradiation intensity of light in each measurement state simply differs in the event that there is one light emitting unit in the optical system. Also, in the event that there are multiple light emitting units in the optical system, this indicates that, of multiple light beams from the multiple light emitting units, the irradiation intensity of at least one light beam differs.

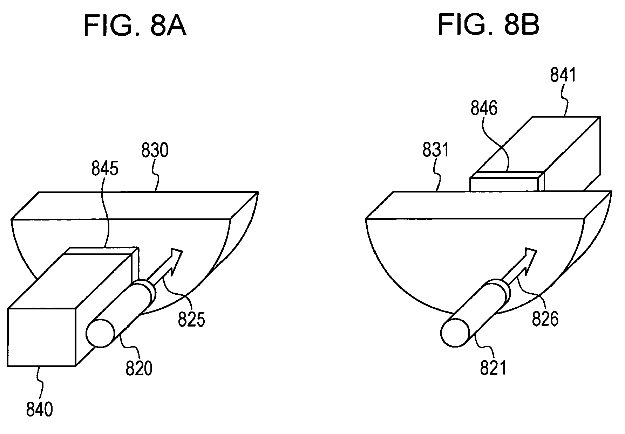

Next, description will be made regarding an example for changing the detection position of an acoustic wave detector serving as a measurement state, with reference to Figs. 8A and 8B.

4. Example for Detecting Photoacoustic Waves in Position Where Detection Surface of Acoustic Wave Detector Differs

A first measurement state illustrated in Fig. 8A is the same as the measurement state illustrated in Fig. 4A.

On the other hand, with a second measurement state illustrated in Fig. 8B, though the irradiation state of light 826 in the second measurement state is the same as the irradiation state of light 825 in the first measurement state, the position of a detection surface 846 of an acoustic wave detector 841 differs from the position of a detection surface 845 of an acoustic wave detector 840 in the first measurement state.

As illustrated in Figs. 8A and 8B, even in the event that the position of the detection surface of the acoustic wave detector differs depending on measurement states, artifacts emerge on a different position depending on measurement states.

This is because whether or not there are multiple reflections which occur by light being irradiated on the surface of an acoustic wave detector, or a propagation path of photoacoustic waves, and so forth depend on the position of the acoustic wave detector. Therefore, appearance positions of artifacts depend on the position of the detection surface of the acoustic wave detector.

Accordingly, with a similarity distribution of multiple optical characteristic value distributions obtained by changing the position of the detection surface of the acoustic wave detector depending on measurement states, similarity of optical absorber images is illustrated high, and similarity of artifacts is illustrated low.

Note that, in the event of having changed an angle made up of the detection surface of the acoustic wave detector and a subject depending on measurement states as well, positions where artifacts appear differ depending on measurement states. In this case, the position of the detection surface of the acoustic wave detector is consequently changed depending on each of the measurement states by changing an angle made up of the detection surface of the acoustic wave detector and a subject surface. That is to say, with the present disclosure, that the position of the detection surface of the acoustic wave detector differs includes that an angle made up of the detection surface of the acoustic wave detector and a subject surface differs.

Note that, with processes in S10 and S40, in the event that light is irradiated on multiple positions, or in the event that light is irradiated from multiple light irradiating units, these light beams do not have to be irradiated at the same time. Specifically, it may be performed to irradiate serial light on each irradiation position, or to irradiate serial light from each irradiating unit.

Also, in the event of irradiating light on multiple different positions, it is desirable to scan the optical system using the optical scanning mechanism. Also, at this time, the optical system may be scanned by the measurement state setting module controlling the optical scanning mechanism.

Also, in the event that the detection surface of the acoustic wave detector detects photoacoustic waves in multiple positions, photoacoustic waves may be detected at multiple positions using multiple acoustic wave detectors, or an acoustic wave detector may be scanned by the acoustic wave detector scanning mechanism to detect photoacoustic waves at multiple positions. Also, at this time, the measurement state setting module may scan the acoustic wave detector by controlling the acoustic wave detector scanning mechanism.

Also, the optical system and acoustic wave detector may be scanned while maintaining a relative position relation between the optical system and the acoustic wave detector.

Also, with the present embodiment, though the measurement state setting module sets the first measurement state and second measurement state by controlling driving of the light source or optical system, a worker may set the light source or optical system so as to realize the first measurement state and second measurement state.

Also, at the time of comparing multiple measurement states, in the event that the number of indications of optical characteristic value distribution with a different measurement state increases, discriminating precision between optical absorber images and artifacts is enhanced, and accordingly, it is desirable to increase the number of measurement states.

S70: Process to Obtain Similarity Distribution between

First Optical Characteristic Value Distribution and Second Optical Characteristic Value Distribution

Next, description will be made in detail regarding a method for obtaining similarity distribution between a first optical characteristic value distribution and a second optical characteristic value distribution illustrated in S70 in Fig. 3.

Here, with the present disclosure, the similarity distribution includes a correlation value distribution based on the first optical characteristic value distribution and the second optical characteristic value distribution, or a distribution based on synthesized data where the first optical characteristic value distribution and second optical characteristic value distribution are synthesized.

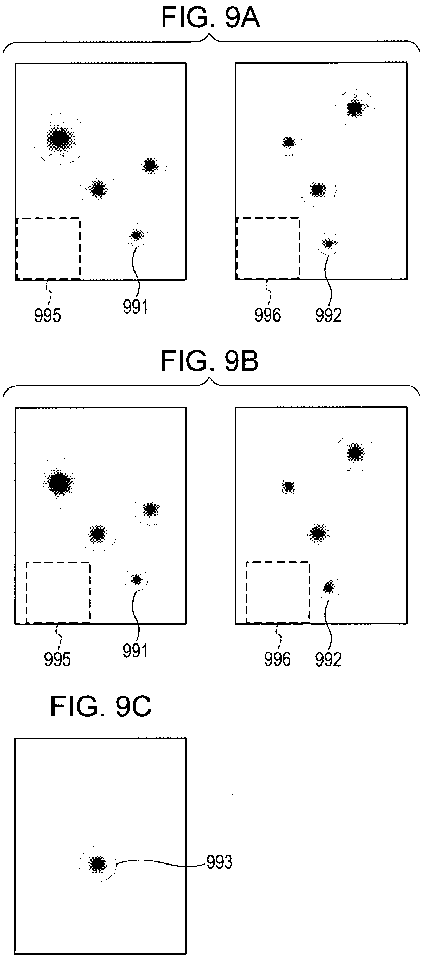

Now, description will be made regarding an example wherein the data processing module 253 calculates a correlation value between a first initial sound pressure distribution serving as the first optical characteristic value distribution and a second initial sound pressure distribution serving as the second optical characteristic value distribution to obtain a similarity distribution based on this correlation value, with reference to Figs. 9A to 9C.

First, the data processing module 253 sets a local region 995 for a first initial sound pressure distribution 991. Next, the data processing module 253 sets a local region 996 in the same position as the local region 995 in the second initial sound pressure distribution 992. Here, in the event of enhancing numeric precision of the correlation value, the local region may be set great. Also, in the event of enhancing position precision of the correlation value, the local region may be set small. Note that the shape of the local region 995 is not restricted to a rectangle, and an optional shape may be employed, a circle may be employed, or a rectangular surrounding portion alone may be set as the local region 995.

Next, the data processing module 253 calculates a correlation value between the initial sound pressure distribution within the local region 995 and the initial sound pressure distribution within the local region 996. The data processing module 253 then obtains similarity in the local regions based on this correlation value.

Here, as a method for calculating the correlation value, ZNCC (Zero-mean Normalized Cross-Correlation) indicated in Expression (1), or a cross-correlation indicated in Expression (2) may be employed. The ZNCC and cross-correlation represent that the higher a correlation value R is, the higher similarity is.

$$R = \frac{\sum_i \sum_j \sum_k \{(P_1(i,j,k) - P_{1a})(P_2(i,j,k) - P_{2a})\}}{\sqrt{\sum_i \sum_j \sum_k (P_1(i,j,k) - P_{1a})^2 \times \sum_i \sum_j \sum_k (P_2(i,j,k) - P_{2a})^2}} \qquad \cdots (1)$$

$$R = \frac{\sum_i \sum_j \sum_k (P_1(i,j,k))(P_2(i,j,k))}{\sqrt{\sum_i \sum_j \sum_k P_1(i,j,k)^2 \times \sum_i \sum_j \sum_k P_2(i,j,k)^2}} \qquad \cdots (2)$$

Here, R denotes the correlation value, and (i, j, k) denotes coordinates in a optical characteristic value distribution. $P_1(i, j, k)$ denotes first initial sound pressure in the coordinates (i, j, k), $P_2(i, j, k)$ denotes second initial sound pressure in the coordinates (i, j, k), $P_{1a}$ denotes a mean value of initial sound pressure within a local region set in the first initial sound pressure distribution, and $P_{2a}$ denotes a mean value of initial sound pressure within a local region set in the second initial sound pressure distribution.

Also, as another method for calculating the correlation value, SSD (Sum of Squared Difference) indicated in Expression (3), or SAD (Sum of Absolute Difference) indicated in Expression (4), or the like may be employed. The SSD and SAD represent that the lower the correlation value R is, the higher similarity is. Therefore, with the SSD and SAD, the inverse number of the correlation value R may be taken as similarity.

$$R = \sum_i \sum_j \sum_k \{I_1(i,j,k) - I_2(i,j,k)\}^2 \qquad \cdots (3)$$

$$R = \sum_i \sum_j \sum_k |I_1(i,j,k) - I_2(i,j,k)| \qquad \cdots (4)$$

Note that a result obtained by weighing the correlation value or the inverse number of the correlation value may be taken as similarity. Also, with Expressions (1) to (4), though there has been indicated the initial sound pressure, other optical characteristic values may be applied to Expressions (1) to (4).

Also, a method for obtaining the correlation value is not restricted to the above-mentioned method, and a method for obtaining a heretofore known correlation value may be employed.

Note that, as a method for obtaining similarity, there may be employed a method for obtaining similarity based on synthesized data synthesized from the first optical characteristic value distribution and the second optical characteristic value distribution. For example, the data processing module 253 may calculate summation, product, and root-mean-square of the overall or a portion between the first optical characteristic value distribution and the second optical characteristic value distribution to obtain results thereof as similarity. Also, a result obtained by weighing this synthesized data may be employed similarity.

Note that the method for obtaining similarity is not restricted to the method for obtaining similarity based on the correlation value or synthesized data. Any method may be employed as long as the method enables similarity to be obtained from multiple optical characteristic value distributions.

Next, the data processing module 253 sets, as illustrated in Fig. 9B, the local regions 995 and 996 in a position different from the position illustrated in Fig. 9A to obtain similarity in the same way as described above.

The data processing module 253 may obtain a similarity distribution 993 of the entire data region as illustrated in Fig. 9C by repeatedly performing the above-mentioned processes on the entire data region.

Here, though it is desirable to move a position to which a local region is set for each voxel, only a region where a detailed distribution is desired may be moved with fine amount of movement after obtaining a similarity distribution by setting amount of movement so as to be great.

Also, a similarity distribution of a portion of the data region may be obtained instead of a similarity distribution of the entire data region.

Note that the signal processing device 250 may perform preprocessing such as blurring processing or enhancement processing or the like on each optical characteristic value distribution before obtaining a similarity distribution.

Here, the blurring processing is processing for blurring an optical characteristic value distribution, which removes high-frequency random noise, and also deteriorates sensitivity for misalignment between measurement states. Specifically, a Gaussian filter, a spatial-frequency low pass filter, a moving average filter, or the like may be employed as the blurring processing.

Also, the enhancement processing is processing for emphasizing a portion matched with a pattern peculiar to optical absorbers in an optical characteristic value distribution. Specifically, the template matching method may be used for an optical characteristic value distribution by taking a pattern peculiar to optical absorbers as a template. Also, the template matching method may be used by taking a pattern peculiar to artifacts or random noise as a template. At this time, there may be performed processing to increase or processing to decrease intensity of an optical characteristic value distribution of a region extracted by the template matching method, or processing to increase or processing to decrease intensity of an optical characteristic value distribution of a region other than a region extracted by the template matching method.

In this manner, the optical characteristic value distribution in each measurement state subjected to the preprocessing is an optical characteristic value distribution where an optical absorber image or artifacts have been enhanced. Therefore, a similarity distribution based on each optical characteristic value distribution subjected to the preprocessing becomes a similarity distribution where an optical absorber image or artifacts are more enhanced as compared to a similarity distribution based on an optical characteristic value distribution not subjected to the preprocessing.

S80: Process to Display Similarity Distribution

Next, an example will be described wherein a similarity distribution illustrated in S80 in Fig. 3 is displayed.

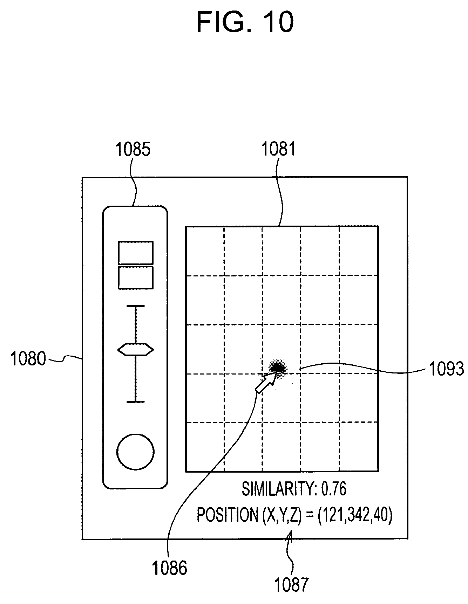

First, description will be made regarding an example wherein a similarity distribution alone is displayed on the display device, with reference to Fig. 10.

The data output module 256 outputs the similarity distribution obtained in S70 on a display device 1080. As illustrated in Fig. 10, a similarity distribution 1093 is then displayed on a display region 1081 of the display device 1080.

Here, an operating unit 1085 may be used for operating a display state such as contrast or the like or a pointer 1086. Also, coordinates of a position specified by the pointer 1086, and similarity in the coordinates thereof may be displayed as numeric information 1087.

Note that, in addition to a similarity distribution, an optical characteristic value distribution in each measurement state may be output to the display device. At this time, multiple display regions may be provided to display a similarity distribution and each optical characteristic value distribution on the display regions, or multiple data may be displayed by being superimposed on one display region.

In the event of displaying multiple data by superimposition, each data may be visibly recognized even when multiple data are overlapped by changing transmittance of each data at the time of display. Also, intensity of data may be displayed with shading of its color by assigning a color to each data. Also, intensity of data may be represented with its color by assigning shading to each data. Also, at this time, it is desirable that setting of transmittance, colors, shading and so forth is performed by the worker at the operating unit provided to the display device so as to be performed in an interactive manner.

As described above, an optical absorber image or artifacts may readily be distinguished by obtaining a similarity distribution based on optical characteristic value distributions obtained in multiple different measurement states.

Second Embodiment

The present embodiment differs from other embodiments in that a light intensity distribution within a subject in each measurement state is displayed in addition to an optical characteristic value distribution obtained in each measurement state. Here, the light intensity distribution includes data to be displayed on the display unit, obtained by performing luminance value conversion on the light intensity distribution.

Incidentally, initial sound pressure PO of photoacoustic waves depends on light intensity $\Phi$ as represented with a relation in Expression (5).

$$PO = \Gamma \cdot \Phi \cdot \mu a \qquad \ldots (5)$$

Here, $\Gamma$ represents a Grueneisen constant, and $\mu a$ represents an optical absorption coefficient. As represented with Expression (5), when light intensity irradiated on an optical absorber has a great value, initial sound pressure of photoacoustic waves to be generated also increases. Specifically, an SN ratio of a detection signal corresponding to a region where much light intensity is irradiated increases. Therefore, an optical characteristic value distribution of a region where much light intensity is irradiated is an optical characteristic value distribution obtained from a detection signal with a great SN ratio, and accordingly, reliability is high. On the contrary, reliability of an optical characteristic value distribution of a region where light is insufficiently irradiated is low. This may be applied to a similarity distribution obtained based on the optical characteristic value distributions in multiple measurement states.

Therefore, with the present embodiment, in addition to a similarity distribution of the optical characteristic value distribution obtained in each measurement state, a light intensity distribution within a subject in each measurement state is obtained, and both are displayed, and accordingly, of the similarity distribution, a region with high reliability may be determined.

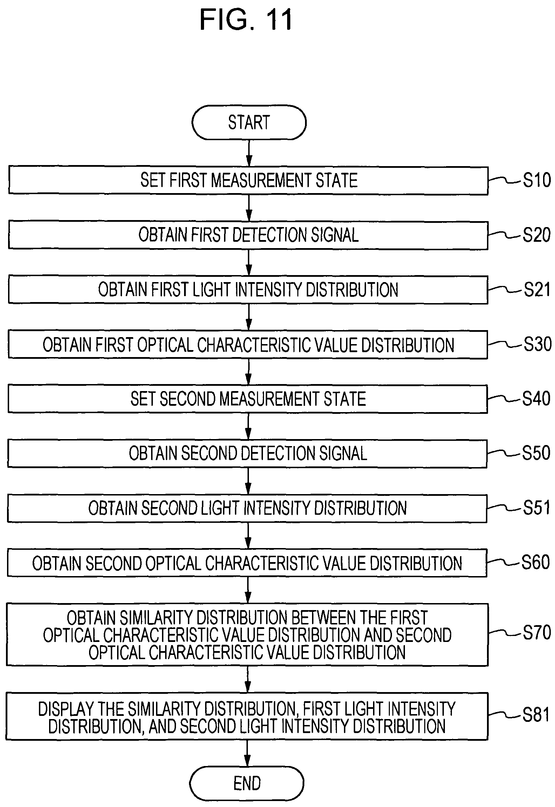

Hereinafter, a method for displaying a light intensity distribution will be described with reference to a flowchart illustrated in Fig. 11. Note that the same processing as with the flowchart illustrated in Fig. 3 will be denoted with the same processing number, and description thereof will be omitted. Also, description will be made using the subject information obtaining device illustrated in Fig. 2.

S21, S51: Process to Obtain Light Intensity Distribution

First, the light intensity distribution obtaining module 254 of the signal processing device 250 obtains a first light intensity distribution based on light in the first measurement state, and obtains a second light intensity distribution based on light in the second measurement state.

Here, as a method for obtaining a light intensity distribution, there may be employed a method for obtaining a light intensity distribution within a subject from a beam profile of irradiation light for the subject by calculation of light propagation within the subject. Also, an arrangement may be made wherein a beam profile of irradiation light for a subject, and a beam profile of light externally emitted from the subject are measured, and a light intensity distribution within the subject is obtained from a relation between both. Note that, in the event that the same beam profile of irradiation light for a subject is employed if irradiation settings are not changed, and accordingly, beam profile data saved in the memory beforehand may be employed instead.

S81: Process to Display Similarity Distribution and Light Intensity Distribution

Next, the data output module 256 outputs the similarity distribution, first light intensity distribution, and second light intensity distribution to the display device 280, and causes the display device 280 to display the similarity distribution, first light intensity distribution, and second light intensity distribution.

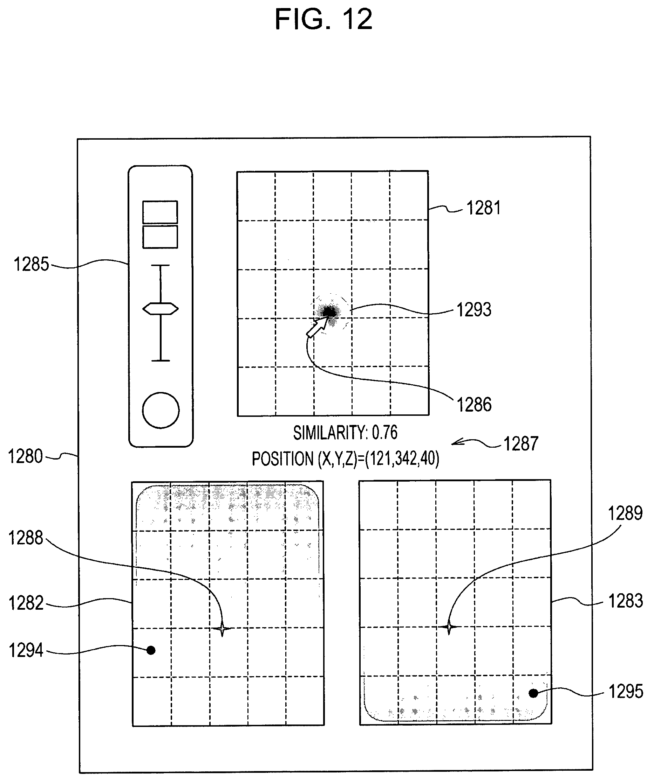

Hereinafter, an example will be described wherein a similarity distribution and a light intensity distribution in each measurement state are output to separate display regions and are displayed in a row respectively, with reference to Fig. 12.

First, the data output module 256 outputs a similarity distribution 1293 to a first display region 1281 of a display device 1280, outputs a first light intensity distribution 1294 to a second display region 1282, and outputs a second light intensity distribution 1295 to a third display region 1283. As illustrated in Fig. 12, the similarity distribution and the light intensity distribution in each measurement state are displayed on the display device 1280 in a row.

Also, with the display device 1280, a first pointer 1286 within the first display region 1281 may be moved using an operating unit 1285 to display coordinates of a position specified by the first pointer 1286 and similarity in the coordinates thereof as numeric information 1287.

In order to facilitate comparison of data displayed in each display region, for example, when specifying certain coordinates within the first display region 1281 using the first pointer 1286, a second pointer 1288 is displayed in a position corresponding to the first pointer 1286 within the second display region 1282. Similarly, a third pointer 1289 is displayed in a position corresponding to the first pointer 1286 within the third display region 1283. Note that an arrangement may also be made wherein certain coordinates are specified by the second pointer 1288 or third pointer 1289, and the corresponding position within another display region is specified.

Also, though it is desirable that the display regions are displayed in the same position, same range, and same dynamic range in conjunction manner, the display regions may individually be adjusted. Also, though it is desirable that the display regions and the operating unit are provided to one display device, multiple display devices to which the display regions and operating unit are provided may also be prepared.

In this manner, the light intensity distributions are displayed in separate display regions, and accordingly, visibility for the light intensity distributions may be enhanced, respectively.

Also, a similarity distribution and the light intensity in each measurement state may be superimposed and displayed on the display device.

Note that, an arrangement may be made wherein in the event that multiple data are superimposed and displayed, transmittance of each data is changed and displayed, and accordingly, each data may be visibly recognized even when the multiple data are overlapped. Also, an arrangement may be made wherein a color is assigned to each data, and intensity of data is displayed with shading of its color. Also, an arrangement may be made wherein shading is assigned to each data, and intensity of data is represented with its color. Also, at this time, it is desirable that setting of transmittance, colors, shading and so forth is performed by the worker at the operating unit provided to the display device so as to be performed in an interactive manner.

Also, the data output module 256 may output, in the same way as with the light intensity distribution described in the present embodiment, a sensitivity distribution of the acoustic wave detector 240 to the display device 280 and causes the display device 280 to display this. An SN ratio of a detection signal corresponding to a region where the sensitivity of the acoustic wave detector 240 is high, and accordingly, a sensitivity distribution of the acoustic wave detector 240 is displayed on the display device in addition to a similarity distribution, and accordingly, of the similarity distribution, a region where reliability is high obtained from a detection signal with a high SN ratio may be determined.

Note that, with the present disclosure, the sensitivity of the acoustic wave detector mentioned here includes attenuation of acoustic waves in a propagation path from the sound source to the acoustic wave detector, and the directivity angle of the acoustic wave detector.

Third Embodiment

The present embodiment differs from other embodiments in that of a light intensity distribution, a confidence region which is a region where light is sufficiently irradiated is obtained. The confidence region mentioned here includes data to be displayed on the display unit obtained by performing luminance value conversion on the confidence region.

With the photoacoustic imaging, it is desirable that of the light intensity distribution obtained in the second embodiment, a region where light is sufficiently irradiated is displayed in an enhanced manner. This is because a detection signal corresponding to a region where light is sufficiently irradiated is high in an SN ratio and high in reliability.

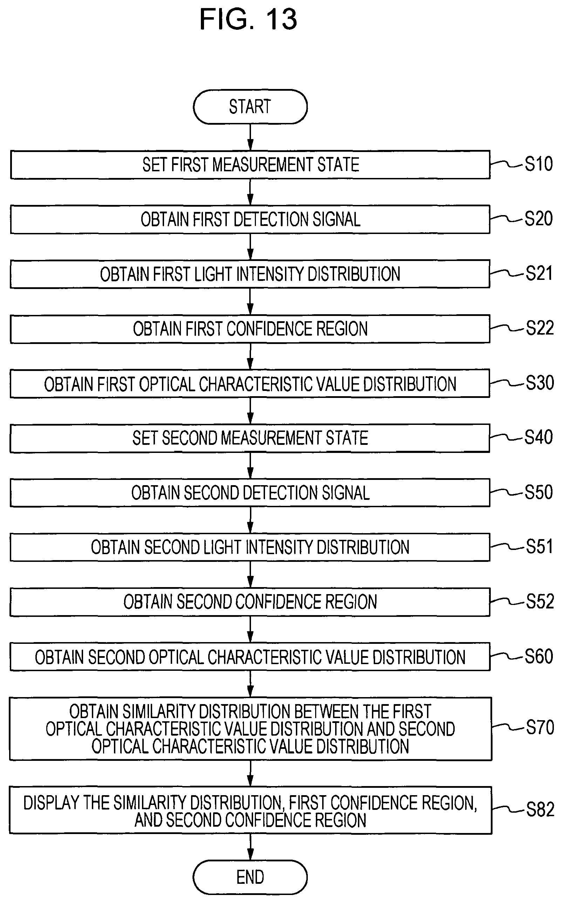

Hereinafter, a subject information obtaining method using a confidence region will be described with reference to the flowchart illustrated in Fig. 13. Note that the same processing as with the flowchart illustrated in Fig. 3 will be denoted with the same processing number, and description thereof will be omitted. Also, description will be made using the subject information obtaining device illustrated in Fig. 2.

S22, S52: Process to Obtain Confidence Region

First, the confidence region obtaining module 255 of the signal processing device 250 sets a threshold whereby it is conceived that a sufficient number of signals are obtained for each of the light intensity distributions. As for the threshold whereby it is conceived that a sufficient number of signals are obtained, it is desirable to set a desired level based on the SN ratio.

Next, the confidence region obtaining module 255 performs processing to increase a light intensity value of a region of which the light intensity value is greater than the threshold, or processing to decrease a light intensity value of a region of which the light intensity value is smaller than the threshold, to obtain a confidence region with a region where light is sufficiently irradiated being enhanced. Specifically, the confidence region obtaining module 255 obtains a first confidence region based on the first light intensity distribution, and obtains a second confidence region based on the second light intensity distribution.

Note that the confidence region obtaining module 255 may obtain a confidence region by binarizing a light intensity distribution with the threshold as a reference.

Also, the confidence region obtaining module 255 may calculate the logic operation AND of the confidence region in each measurement state to take a result thereof as a confidence region.

S82: Process to Display Similarity Distribution and Confidence Region

Next, the data output module 256 outputs the confidence region obtained in S22 or S52 to the display device 280, and causes the display device 280 to display the confidence region. With the present process, the confidence region may be displayed by being superimposed on the above-mentioned similarity distribution, optical characteristic value distribution, and light intensity distribution, or may be displayed in a row with the similarity distribution, optical characteristic value distribution, and light intensity distribution.

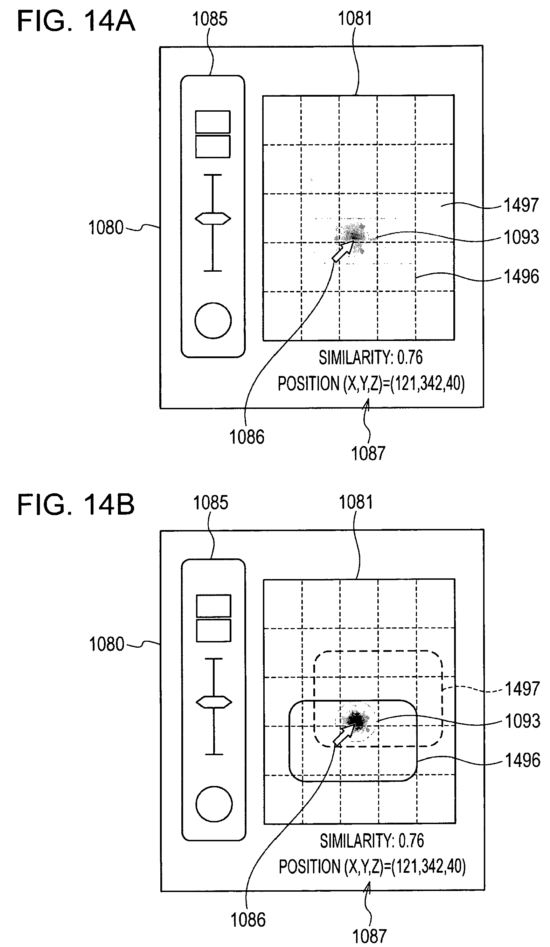

With the present embodiment, as an example thereof, an example will be described wherein the confidence region is displayed by being superimposed on the similarity distribution illustrated in Fig. 10, with reference to Figs. 14A and 14B. However, in Figs. 14A and 14B, the same configuration as with Fig. 10 is denoted with the same reference numeral. Here, as a confidence region illustrated in Figs. 14A and 14B, there is employed a confidence region where a threshold is set for the light intensity distribution in each measurement state which have been represented by being binarized.

First, the data output module 256 outputs the similarity distribution, first confidence region, and second confidence region to a display region 1081 of a display device 1080. As illustrated in Fig. 14A, the similarity distribution 1091, first confidence region 1496, and second confidence region 1497 are superimposed and displayed on the display region 1081.

Note that, as illustrated in Fig. 14A, in the event of superimposing and displaying the similarity distribution and confidence regions, it is desirable to display the similarity distribution and confidence regions by changing the transmittance of the similarity distribution or confidence regions. Also, it is desirable to display the similarity distribution and confidence regions with different colors. Further, with regard to the confidence regions, it is desirable to change their colors for each measurement state.

Also, in the event of having obtained confidence regions by binarizing a light intensity distribution, as illustrated in Fig. 14B, the outer circumferences of the confidence regions may be surrounded with a line. However, even in the event of having obtained confidence regions without binarizing, predetermined values in the confidence regions may be connected with a line to obtain the outer circumferences of the confidence regions.

In this manner, in addition to a similarity distribution, a confidence region where light is sufficiently irradiated is obtained, and both thereof are displayed, and accordingly, of the similarity distribution, a region with high similarity of reliability may readily be determined.

Also, the confidence region obtaining module 255 may obtain, in the same way as with the confidence region based on a light intensity distribution described in the present embodiment, a confidence region based on the sensitivity distribution of the acoustic wave detector 240. In this case, the data output module 256 may output the confidence region based on the sensitivity distribution of the acoustic wave detector 240 to the display device 280, and causes the display device 280 to display this.

The confidence region based on the sensitivity distribution of the acoustic wave detector 240 is an optical characteristic value distribution where a region with high sensitivity of the acoustic wave detector 240 is enhanced. Therefore, the confidence region based on the sensitivity of the acoustic wave detector 240 also indicates, as with the confidence region based on a light intensity distribution, a region obtained from a detection signal with a high SN ratio.

Accordingly, in addition to a similarity distribution, confidence regions based on the sensitivity distribution of the acoustic wave detector 240 are displayed on the display device 280, and accordingly, of the similarity distribution, a region with high reliability obtained from a detection signal with a high SN ratio may readily be distinguished.

Fourth Embodiment

With the present embodiment, it is difference with other embodiments to obtain a similarity distribution based on a detection signal obtained by the acoustic wave detector and the confidence region obtained in the third embodiment.

With the present embodiment, the optical characteristic value distribution obtaining module 252 obtains an optical characteristic value distribution where a region with high reliability is enhanced by weighing a confidence region for an optical characteristic value distribution obtained based on a detection signal. An optical characteristic value distribution where a region with high reliability is enhanced is obtained in each measurement state, and a similarity distribution of the optional characteristic value distribution in each measurement state is obtained. The similarity distribution thus obtained represents a distribution of similarity between optical characteristic value distributions with high reliability, and accordingly, reliability of similarity is high.

Note that the optical characteristic value distribution obtaining module 252 may obtain a similarity distribution where a region with high reliability is enhanced by weighing the similarity distribution obtained in the first embodiment using a confidence region.

Also, a detection signal in each measurement state may be weighed with a confidence region. In this case, signal intensity of detection time corresponding to a confidence region is weighed with the confidence region. Based on a detection signal weighed with the confidence region, an optical characteristic value distribution where a region with high reliability is enhanced is then obtained. Regarding each measurement state, a similarity distribution of the optical characteristic value distributions obtained by performing this process may be obtained. In this manner, a similarity distribution based on a detection signal weighed with a confidence region also becomes a similarity distribution where a region with high reliability is enhanced.

However, in the event that confidence regions have been binarized, only signal intensity of detection time corresponding to a confidence region with a low value may be reduced. Also, even in the event that confidence regions have not been binarized, signal intensity of detection time corresponding to a confidence region with a predetermined value or less may be reduced.

Now, as a weighing method, a method to perform multiplication between an optical characteristic value distribution or similarity distribution and a confidence region may be employed, for example. Note that processing other than multiplication may be performed as long as a similarity distribution where a region with high reliability is enhanced may be obtained by the method.

Hereinafter, an example of the subject information obtaining method according to the present embodiment will be described using the subject information obtaining device illustrated in Fig. 2.

The optical characteristic value distribution obtaining module 252 obtains a first initial sound pressure distribution by performing reconstitution processing on a first detection signal. The optical characteristic value distribution obtaining module 252 multiplies this first initial sound pressure distribution, and a first confidence region based on a first light intensity distribution, thereby obtaining the first initial sound pressure distribution where a region with high reliability is enhanced.

Also, similarly, the optical characteristic value distribution obtaining module 252 obtains a second initial sound pressure distribution by performing reconstruction processing on a second detection signal. The optical characteristic value distribution obtaining module 252 then multiplies this second initial sound pressure distribution and a second confidence region based on a second light intensity distribution, thereby obtaining the second initial sound pressure distribution where a region with high reliability is enhanced.

Next, the data processing module 253 obtains a similarity distribution between the first initial sound pressure distribution where a region with high reliability is enhanced and the second initial sound pressure distribution. The similarity distribution thus obtained is a similarity distribution where a region with high reliability is enhanced.

The data output module 256 then outputs the similarity distribution where a region with high reliability is enhanced to the display device 280, and the similarity distribution where a region with high reliability is enhanced is displayed on the display device 280.

The similarity distribution thus displayed is a distribution where similarity of a region with light intensity being sufficiently irradiated or a region with high sensitivity of the acoustic wave detector is enhanced, and accordingly, only a similarity distribution with high reliability may be observed.

Fifth Embodiment

Majority of function information obtained by the photoacoustic imaging is information relating to an optical absorption coefficient, and accordingly, it is desirable to obtain information relating to an optical absorption coefficient as an optical characteristic value distribution.

Therefore, with the present embodiment, information relating to an optical absorption coefficient distribution obtained by performing light intensity correction on an initial sound pressure distribution or optical energy density distribution is handled as an optical characteristic value distribution. That is to say, with the present embodiment, examples of the optical characteristic value distributions include an optical absorption coefficient distribution and an oxygen saturation distribution.

Hereinafter, an example of a subject information obtaining method according to the present embodiment will be described with reference to the flowchart illustrated in Fig. 11. Note that, with regard to its configuration, description will be made with reference to the configuration of the subject information obtaining device illustrated in Fig. 2.

First, in S30 according to the present embodiment, first, the optical characteristic value distribution obtaining module 252 obtains, based on Expression (5), a first optical absorption coefficient distribution serving as a fist optical characteristic value distribution using the first detection signal obtained in S20 and the first light intensity distribution obtained in S21.

Also, similarly, in S60 according to the present embodiment, the optical characteristic value distribution obtaining module 252 obtains a second optical absorption coefficient distribution serving as a second optical characteristic value distribution using the second detection signal obtained in S50 and the second light intensity distribution obtained in S51.

Next, the data processing module 253 obtains a similarity distribution between the first optical absorption coefficient distribution and the second optical absorption coefficient distribution.

The data output module 256 then outputs the similarity distribution to the display device 280, and the similarity distribution is displayed on the display device 280.

With an optical absorption coefficient distribution with an initial sound pressure distribution thus subjected to light intensity correction, not only the position of an optical absorber image in each of the measurement states is the same, but also intensity of an optical absorber image is the same in each of the measurement states.

On the other hand, artifacts are failed to be removed even when performing light intensity correction thereon, and accordingly emerge in different positions depending on the measurement states.

Accordingly, with a similarity distribution between optical characteristic value distributions of information subjected to light intensity correction in a different measurement state as with the present embodiment, similarity of optical absorber images which exit with the same intensity in the same position is illustrated high. Therefore, the optical absorber images may readily be distinguished.

Note that, as with oxygen saturation or the like, in the event of taking optical characteristic values obtained by multiple times of measurement as an optical characteristic value distribution, multiple times of measurement for obtaining a first oxygen saturation distribution serving as a first optical characteristic value distribution is taken as measurement in a first measurement state. An arrangement may be made wherein different multiple times of measurement is taken as measurement in a second measurement state, and a second oxygen saturation distribution serving as a second optical characteristic value distribution is obtained. Here, different multiple times of measurement mentioned here indicates measurement wherein of multiple measurements, the measurement state of at least one measurement has been changed.

Hereinafter, the basic configuration of the subject information obtaining device illustrated in Fig. 2 will be described.

Light Source 210

The light source 210 is a device to generate pulsed light. In order to obtain large output, a laser is desirable as the light source 210, but may be a light-emitting diode or the like. In order to effectively generate photoacoustic waves, light has to be irradiated in a sufficiently short period of time according to a thermal property of the subject 230. In the event that the subject 230 is a living body, it is desirable to set 10 nanoseconds or less as the pulse width of pulsed light generated from the light source 210. Also, the wavelength of pulsed light is a near-infrared region called as a window of a living body, and is preferably around 700 nm to 1200 nm. Light within this region may reach a relatively deep portion of the living body, and accordingly, information of the deep portion may be obtained. Further, with regard to the wavelength of pulsed light, it is desirable that an optical absorption coefficient is high for an object to be observed. Optical System 220

The optical system 220 is a device to guide pulsed light generated at the light source 210 to the subject 230. The optical system 220 is specifically an optical apparatus such as an optical fiber, lens, mirror, diffuser plate, or the like.

With the present disclosure, at the time of multiple times of measurement, the measurement state such as the irradiation shape of pulsed light, light density, irradiation direction for a subject, or the like may be changed using such an optical apparatus. Also, these may be adjusted at the light source 210.

Also, in order to obtain data in a wide range, the optical system 220 may be scanned by the optical scanning mechanism 221 configured so as to scan the optical system 220 to scan the irradiation position of pulsed light. At this time, scanning may be performed in conjunction with the acoustic wave detector 240.

Also, the optical system 220 is not restricted to mentioned above, and any may be employed as long as this satisfies such a function.

Subject 230

The subject 230 becomes an object to be measured. As the subject 230, a living body or a phantom which has simulated a living body, or the like may be employed.

For example, in the event that the subject 230 is a living body, with the subject information obtaining device according to the present disclosure, a blood vessel or the like serving as an optical absorber existing within the subject 230 may be imaged. Also, examples of an optical absorber include hemoglobin, water, melanin, collagen, lipid, and so forth which have a relatively great optical absorber coefficient within a living body, and a living body tissue configured of these.

Also, in the event of a phantom, a substance which has simulated optical characteristics of the above-mentioned optical absorbers may be sealed in the phantom. Acoustic Wave Detector 240

The acoustic wave detector 240 detects photoacoustic waves, and converts these into electric signals.

In order to detect photoacoustic waves in multiple positions, the acoustic wave detector scanning mechanism 241 which is configured so as to scan the acoustic wave detector 240 may scan a single acoustic wave detector to move to multiple positions, or multiple acoustic wave detectors may be installed in different locations.

Also, with the photoacoustic imaging, the acoustic wave detector 240 receives photoacoustic waves generated from the inside of the subject 230, and accordingly, in order to suppress reflection or attenuation of generated photoacoustic waves, the acoustic wave detector 240 has to be installed so as to acoustically be connected to the subject 230. For example, an acoustic matching material such as acoustic matching GEL, water, oil, or the like may be provided between the acoustic wave detector 240 and the subject 230.

Also, as the acoustic wave detector 240, a device with high sensitivity and wide frequency band is desirable, and specific examples thereof include a PZT, PVDF, cMUT, and acoustic wave detector using a Fabr-Perot interferometer. However, the acoustic wave detector 240 is not restricted to that mentioned above, and any may be employed as long as this satisfies the function.

Signal Processing Device 250

The signal processing device 250 performs amplification or conversion into digital signals, or the like regarding electric signals obtained at the acoustic wave detector 240. The converted digital signals are then processed to obtain a similarity distribution, and the data is output to the display device 280. The signal processing device 250 includes an AD converter (ADC), the measurement state setting module 251, optical characteristic value distribution obtaining module 252, data processing module 253, light intensity distribution obtaining module 254, confidence region obtaining module 255, data output module 256, and so forth.

Note that the modules which the signal processing device 250 according to the present embodiment includes may be provided as stand-alone devices, respectively.

Also, in the event of configuring the modules as hardware, the modules are able to be configured as FPGA, ASIC, or the like. Also, each of the modules may be configured as a program causing the computer to execute each of the processes.

Specific examples of the signal processing device include a computer. In order to effectively obtain data, it is desirable to include AD converters (ADC) of which the number is the same as the number of reception elements of the acoustic wave detector 240, but one ADC may be used by sequentially changing a reception element to be connected. Memory 260

The memory 260 is configured to hold an optical characteristic value distribution processed by the signal processing device 250. An optical characteristic value distribution obtained in a different measurement state is held in the memory. It is desirable to prepare memory by the number of measurement states.

Note that, though the memory is to temporarily hold data, and to enable a flexible display method to be performed, the signal processing device 250 may directly transmit data to the display device 280 without employing the memory.

Note that the signal processing device 250 may include the memory 260, or the display device 280 may include the memory 260.

Display Device 280

The display device 280 includes a display region where data is displayed. Also, the display device 280 may include multiple display regions. Note that, with the present disclosure, the display unit means a single display device or multiple display devices.

Also, it is desirable that the display device 280 includes an operating unit to be used for operating a display state or pointer. Further, it is desirable that the single operating unit is provided to each of the display regions. Also, the operating unit may be operated by touch panel operations or by hardware operations such as a mechanical switch or the like. Note that the operating unit may be provided to a device other than the display device 280, e.g., the signal processing device 250. Also, the operating unit may be a stand-alone device.

Also, though it is desirable that the multiple display regions are displayed in the same location, same range, and same dynamic range in an interlocking manner, the multiple display regions may individually be adjusted. Image processing used for such adjustment may be performed by the signal processing device 250.

Also, the signal processing device 250 and display device 280 may be provided in an integral manner. Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments but defined by the following claims.

## Claims

1. A subject information obtaining device comprising:

   a light source (210) arranged to generate light;
   an optical system (220) arranged to emit the light on a subject (230) as light in a first measurement state to generate first photoacoustic waves, and as light in a second measurement state to generate second photoacoustic waves;
   an acoustic wave detector (240) arranged to obtain a first detection signal by detecting the first photoacoustic waves, and to obtain a second detection signal by detecting the second photoacoustic waves;
   signal processing means (250) arranged to obtain a first optical characteristic value distribution within the subject based on the first detection signal, to obtain a second optical characteristic value distribution within the subject based on the second detection signal, and to obtain a similarity value distribution between the first optical characteristic value distribution and the second optical characteristic value distribution; the device **characterised in** further comprising:

   measurement state setting means (250) arranged to set the first measurement state and the second measurement state to be different in position relation between irradiation light, the acoustic wave detector, and the subject.

2. The subject information obtaining device according to Claim 1,
   wherein the measurement state setting means (250) is arranged to set the first measurement state and the second measurement state to be different in:

   irradiation positions of light for a surface of the subject (230), angles made up of a surface of the subject (230) and irradiation directions of light, irradiation intensities of light for a surface of the subject (230), or positions of detection surfaces of the acoustic wave detector (140).

3. The subject information obtaining device according to Claim 1 or 2,

   wherein the signal processing means (250) is arranged to obtain a first light intensity value distribution within the subject (230) when irradiating the subject with light in the first measurement state,
   to obtain a second light intensity value distribution within the subject (230) when irradiating the subject with light in the second measurement state,
   to obtain the first optical characteristic value distribution based on the first detection signal and the first light intensity value distribution, and
   to obtain the second optical characteristic value distribution based on the second detection signal and the second light intensity value distribution.

4. The subject information obtaining device according to Claim 3,

   wherein the signal processing means (250) is arranged to obtain a first initial sound pressure distribution within the subject (230) based on the first plurality of detection signals,
   to obtain a second initial sound pressure distribution within the subject (230) based on the second plurality of detection signals,
   to obtain the first optical characteristic value distribution based on the first initial sound pressure distribution and the first light intensity value distribution, and
   to obtain the second optical characteristic value distribution based on the second initial sound pressure distribution and the second light intensity value distribution.

**5.** The subject information obtaining device according to any one of Claims 1 to 4,

wherein the signal processing means (250) is arranged to obtain a first light intensity value distribution within the subject (230) when irradiating the subject with light in the first measurement state,
to obtain a second light intensity value distribution within the subject (230) when irradiating the subject with light in the second measurement state,
to obtain a first confidence region where the light intensity value of the first light intensity value distribution is greater than a threshold,
to obtain a second confidence region where the light intensity value of the second light intensity value distribution is greater than a threshold, and
to obtain the similarity value distribution based on the first optical characteristic value distribution, the second optical characteristic value distribution and the first confidence region, and the second confidence region.

**6.** The subject information obtaining device according to any one of Claims 1 to 4,

wherein the signal processing means (250) is arranged to obtain a first light intensity value distribution within the subject (230) when irradiating the subject with light in the first measurement state,
to obtain a first confidence region where the light intensity value of the first light intensity value distribution is greater than a threshold, and
to obtain the similarity value distribution based on the first optical characteristic value distribution, the second optical characteristic value distribution, and the first confidence region.

**7.** The subject information obtaining device according to any one of Claims 1 to 6,
wherein the signal processing means (250) is arranged to obtain the similarity value distribution based on a distribution of correlation values between the first optical characteristic value distribution and the second optical characteristic value distribution.

**8.** The subject information obtaining device according to any one of Claims 1 to 7, further comprising a display means (280), wherein the signal processing means (250) is arranged to display the similarity value distribution on the display means (280).

**9.** The subject information obtaining device according to Claim 8,

wherein the signal processing means (250) is arranged to obtain a first light intensity value distribution within the subject (230) when irradiating the subject with light in the first measurement state,
to obtain a second light intensity value distribution within the subject (230) when irradiating the subject with light in the second measurement state,
to obtain a first confidence region where the light intensity value of the first light intensity value distribution is greater than a threshold,
to obtain a second confidence region where the light intensity value of the second light intensity value distribution is greater than a threshold, and
to display the similarity value distribution and the first confidence region and the second confidence region on the display means (280).

**10.** The subject information obtaining device according to Claim 8,

wherein the signal processing means (250) is arranged to obtain a first light intensity value distribution within the subject (230) when irradiating the subject with light in the first measurement state,
to obtain a first confidence region where the light intensity value of the first light intensity value distribution is greater than a threshold, and
to display the similarity value distribution and the first confidence region (280).

**11.** The subject information obtaining device according to any one of Claims 1 to 10,

wherein the acoustic wave detector comprises multiple acoustic wave detectors at multiple positions.

**12.** The subject information obtaining device according to any one of Claims 1 to 10, further comprising an acoustic wave detector scanning means (241) configured to scan the acoustic wave detector.

**13.** A subject information obtaining method comprising:

a process (S30) to obtain a first optical characteristic value distribution within a subject (230) based on a first detection signal obtained by detecting (S20) photoacoustic waves generated by irradiating the subject with light (S10) in a first measurement state;
a process (S60) to obtain a second optical characteristic value distribution within the subject (230) based on a second detection signal obtained by detecting (S50) photoacoustic waves generated by irradiating the subject with light (S40) in a second measurement state; and
a process (S70) to obtain a similarity value distribution between the first optical characteristic value distribution and the second optical characteristic value distribution;

wherein the first measurement state and the second measurement state are set (S10, S40) to be different in position relation between irradiation light, an acoustic wave detector (240) used for detecting the photoacoustic waves, and the subject.

**14.** A program which when running on a computer controlling the device according to claim 1 causes the device according to claim 1 to execute the subject information obtaining method according to Claim 13.

**Patentansprüche**

**1.** Subjektinformationserhaltungsvorrichtung, umfassend:

eine Lichtquelle (210), ausgebildet zum Erzeugen von Licht;
ein optisches System (220), ausgebildet zum Emittieren des Lichts auf ein Subjekt (230) als Licht in einem ersten Messzustand zum Erzeugen erster fotoakustischer Wellen und als Licht in einem zweiten Messzustand zum Erzeugen zweiter fotoakustischer Wellen;
einen Akustikwellendetektor (240), ausgebildet zum Erhalten eines ersten Detektionssignals durch Detektieren der ersten fotoakustischen Wellen und zum Erhalten eines zweiten Detektionssignals durch Detektieren der zweiten fotoakustischen Wellen;
eine Signalverarbeitungseinrichtung (250), ausgebildet zum Erhalten einer ersten Verteilung optischer charakteristischer Werte innerhalb des Subjekts basierend auf dem ersten Detektionssignal, zum Erhalten einer zweiten Verteilung optischer charakteristischer Werte innerhalb des Subjekts basierend auf dem zweiten Detektionssignal, und zum Erhalten einer Verteilung ähnlicher Werte zwischen der ersten Verteilung optischer charakteristischer Werte und der zweiten Verteilung optischer charakteristischer Werte; **dadurch gekennzeichnet, dass** die Vorrichtung ferner umfasst:

eine Messzustandseinstelleinrichtung (250), ausgebildet zum Einstellen des ersten Messzustands und des zweiten Messzustands, damit sich diese hinsichtlich des Positionsverhältnisses zwischen Bestrahlungslicht, dem Akustikwellendetektor und dem Subjekt unterscheiden.

**2.** Subjektinformationserhaltungsvorrichtung nach Anspruch 1,
wobei die Messzustandseinstelleinrichtung (250) ausgebildet ist zum Einstellen des ersten Messzustands und des zweiten Messzustands, damit sich diese unterscheiden hinsichtlich:

Bestrahlungspositionen von Licht für eine Fläche des Subjekts (230), Winkeln zwischen einer Fläche des Subjekts (230) und Bestrahlungsrichtungen von Licht, Bestrahlungsintensitäten von Licht für eine Fläche des Subjekts (230) oder Positionen von Detektionsflächen des Akustikwellendetektors (140).

**3.** Subjektinformationserhaltungsvorrichtung nach Anspruch 1 oder 2,

wobei die Signalverarbeitungseinrichtung (250) ausgebildet ist zum Erhalten einer ersten Verteilung von Lichtintensitätswerten innerhalb des Subjekts (230), wenn das Subjekt im ersten Messzustand mit Licht bestrahlt wird, zum Erhalten einer zweiten Verteilung von Lichtintensitätswerten innerhalb des Subjekts (230), wenn das Subjekt im zweiten Messzustand mit Licht bestrahlt wird,
zum Erhalten der ersten Verteilung optischer charakteristischer Werte basierend auf dem ersten Detektionssignal und der ersten Verteilung von Lichtintensitätswerten, und
zum Erhalten der zweiten Verteilung optischer charakteristischer Werte basierend auf dem zweiten Detektionssignal und der zweiten Verteilung von Lichtintensitätswerten.

4. Subjektinformationserhaltungsvorrichtung nach Anspruch 3,

wobei die Signalverarbeitungseinrichtung (250) ausgebildet ist zum Erhalten einer ersten Anfangsschalldruckverteilung innerhalb des Subjekts (230) basierend auf den ersten mehreren Detektionssignalen,
zum Erhalten einer zweiten Anfangsschalldruckverteilung innerhalb des Subjekts (230) basierend auf den zweiten mehreren Detektionssignalen,
zum Erhalten der ersten Verteilung optischer charakteristischer Werte basierend auf der ersten Anfangsschalldruckverteilung und der ersten Verteilung von Lichtintensitätswerten, und
zum Erhalten der zweiten Verteilung optischer charakteristischer Werte basierend auf der zweiten Anfangsschalldruckverteilung und der zweiten Verteilung von Lichtintensitätswerten.

5. Subjektinformationserhaltungsvorrichtung nach einem der Ansprüche 1 bis 4,

wobei die Signalverarbeitungseinrichtung (250) ausgebildet ist zum Erhalten einer ersten Verteilung von Lichtintensitätswerten innerhalb des Subjekts (230), wenn das Subjekt im ersten Messzustand mit Licht bestrahlt wird,
zum Erhalten einer zweiten Verteilung von Lichtintensitätswerten innerhalb des Subjekts (230), wenn das Subjekt im zweiten Messzustand mit Licht bestrahlt wird,
zum Erhalten eines ersten Konfidenzbereichs, wo der erste Lichtintensitätswert der ersten Verteilung von Lichtintensitätswerten höher ist als ein Schwellenwert,
zum Erhalten eines zweiten Konfidenzbereichs, wo der Lichtintensitätswert der zweiten Verteilung von Lichtintensitätswerten höher ist als ein Schwellenwert, und
zum Erhalten der Verteilung ähnlicher Werte basierend auf der ersten Verteilung optischer charakteristischer Werte, der zweiten Verteilung optischer charakteristischer Werte und dem ersten Konfidenzbereich und dem zweiten Konfidenzbereich.

6. Subjektinformationserhaltungsvorrichtung nach einem der Ansprüche 1 bis 4,

wobei die Signalverarbeitungseinrichtung (250) ausgebildet ist zum Erhalten einer ersten Verteilung von Lichtintensitätswerten innerhalb des Subjekts (230), wenn das Subjekt im ersten Messzustand mit Licht bestrahlt wird,
zum Erhalten eines ersten Konfidenzbereichs, wo der Lichtintensitätswert der ersten Verteilung von Lichtintensitätswerten höher ist als ein Schwellenwert, und
zum Erhalten der Verteilung ähnlicher Werte basierend auf der ersten Verteilung optischer charakteristischer Werte, der zweiten Verteilung optischer charakteristischer Werte und dem ersten Konfidenzbereich.

7. Subjektinformationserhaltungsvorrichtung nach einem der Ansprüche 1 bis 6, wobei die Signalverarbeitungseinrichtung (250) ausgebildet ist zum Erhalten der Verteilung ähnlicher Werte basierend auf einer Verteilung von Korrelationswerten zwischen der ersten Verteilung optischer charakteristischer Werte und der zweiten Verteilung optischer charakteristischer Werte.

8. Subjektinformationserhaltungsvorrichtung nach einem der Ansprüche 1 bis 7, ferner umfassend eine Anzeigeeinrichtung (280), wobei die Signalverarbeitungseinrichtung (250) ausgebildet ist zum Anzeigen der Verteilung ähnlicher Werte auf der Anzeigeeinrichtung (280).

9. Subjektinformationserhaltungsvorrichtung nach Anspruch 8, wobei die Signalverarbeitungseinrichtung (250) ausgebildet ist, zum Erhalten einer ersten Verteilung von Lichtintensitätswerten innerhalb des Subjekts (230), wenn das Subjekt im ersten Messzustand mit Licht bestrahlt wird,

zum Erhalten einer zweiten Verteilung von Lichtintensitätswerten innerhalb des Subjekts (230), wenn das Subjekt im zweiten Messzustand mit Licht bestrahlt wird,
zum Erhalten eines ersten Konfidenzbereichs, wo der Lichtintensitätswert der ersten Verteilung von Lichtintensitätswerten höher ist als ein Schwellenwert,
zum Erhalten eines zweiten Konfidenzbereichs, wo der Lichtintensitätswert der zweiten Verteilung von Lichtintensitätswerten höher ist als ein Schwellenwert, und
zum Anzeigen der Verteilung ähnlicher Werte, des ersten Konfidenzbereichs und des zweiten Konfidenzbereichs auf der Anzeigeeinrichtung (280).

**10.** Subjektinformationserhaltungsvorrichtung nach Anspruch 8, wobei die Signalverarbeitungseinrichtung (250) ausgebildet ist zum Erhalten einer ersten Verteilung von Lichtintensitätswerten innerhalb des Subjekts (230), wenn das Subjekt im ersten Messzustand mit Licht bestrahlt wird,

zum Erhalten eines ersten Konfidenzbereichs, wo der Lichtintensitätswert der ersten Verteilung von Lichtintensitätswerten höher ist als ein Schwellenwert, und
zum Anzeigen der Verteilung ähnlicher Werte und des ersten Konfidenzbereichs (280).

**11.** Subjektinformationserhaltungsvorrichtung nach einem der Ansprüche 1 bis 10, wobei der Akustikwellendetektor mehrere Akustikwellendetektoren an mehreren Positionen umfasst.

**12.** Subjektinformationserhaltungsvorrichtung nach einem der Ansprüche 1 bis 10, ferner umfassend eine Akustikwellendetektorscaneinrichtung (241), konfiguriert zum Scannen des Akustikwellendetektors.

**13.** Subjektinformationserhaltungsverfahren, umfassend:

einen Vorgang (S30) zum Erhalten einer ersten Verteilung optischer charakteristischer Werte innerhalb eines Subjekts (230) basierend auf einem ersten Detektionssignal, erhalten durch Detektieren (S20) von durch Bestrahlen des Subjekts mit Licht (S10) in einem ersten Messzustand erzeugter fotoakustischer Wellen;
einen Vorgang (S60) zum Erhalten einer zweiten Verteilung optischer charakteristischer Werte innerhalb des Subjekts (230) basierend auf einem zweiten Detektionssignal, erhalten durch Detektieren (S50) von durch Bestrahlen des Subjekts mit Licht (S40) in einem zweiten Messzustand erzeugter fotoakustischer Wellen; und
einen Vorgang (S70) zum Erhalten einer Verteilung ähnlicher Werte zwischen der ersten Verteilung optischer charakteristischer Werte und der zweiten Verteilung optischer charakteristischer Werte;

wobei der erste Messzustand und der zweite Messzustand eingestellt sind (S10, S40), sich durch das Positionsverhältnis zwischen Bestrahlungslicht, einem zum Detektieren der fotoakustischen Wellen verwendeten Akustikwellendetektor (240) und dem Subjekt zu unterscheiden.

**14.** Programm, das bei Ausführung auf einem die Vorrichtung nach Anspruch 1 steuernden Computer die Vorrichtung nach Anspruch 1 dazu veranlasst, das Subjektinformationserhaltungsverfahren nach Anspruch 13 auszuführen.

**Revendications**

**1.** Dispositif d'obtention d'informations d'un sujet comprenant :

une source de lumière (210) conçue pour générer de la lumière ;
un système optique (220) conçu pour émettre la lumière sur un sujet (230) sous la forme d'une lumière se trouvant dans un premier état de mesure afin de générer des premières ondes photoacoustiques, et sous la forme d'une lumière se trouvant dans un second état de mesure afin de générer des secondes ondes photoacoustiques ;
un détecteur d'ondes acoustiques (240) conçu pour obtenir un premier signal de détection en détectant les premières ondes photoacoustiques, et pour obtenir un second signal de détection en détectant les secondes ondes photoacoustiques ;
un moyen de traitement du signal (250) conçu pour obtenir une première distribution de valeurs caractéristiques optiques au sein du sujet sur la base du premier signal de détection, pour obtenir une seconde distribution de valeurs caractéristiques optiques au sein du sujet sur la base du second signal de détection, et pour obtenir une distribution de valeurs de ressemblance entre la première distribution de valeurs caractéristiques optiques et la seconde distribution de valeurs caractéristiques optiques ;

le dispositif étant **caractérisé en ce qu'**il comprend en outre :

un moyen de réglage d'état de mesure (250) conçu pour régler le premier état de mesure et le second état de mesure afin qu'ils soient différents en ce qui concerne la relation de position entre la lumière d'exposition, le détecteur d'ondes acoustiques, et le sujet.

**2.** Dispositif d'obtention d'informations d'un sujet selon la revendication 1, dans lequel le moyen de réglage d'état de mesure (250) est conçu pour régler le premier état de mesure et le second état de mesure afin qu'ils soient différents en ce qui concerne :

les positions d'exposition de la lumière pour une surface du sujet (230), les angles formés par une surface du sujet (230) et les directions d'exposition de la lumière, les intensités d'exposition de la lumière pour une surface du sujet (230), ou les positions de surfaces de détection du détecteur d'ondes acoustiques (140).

3. Dispositif d'obtention d'informations d'un sujet selon la revendication 1 ou 2,
dans lequel le moyen de traitement du signal (250) est conçu pour obtenir une première distribution de valeurs d'intensité de la lumière au sein du sujet (230) lors de l'exposition du sujet à la lumière se trouvant dans le premier état de mesure,
pour obtenir une seconde distribution de valeurs d'intensité de la lumière au sein du sujet (230) lors de l'exposition du sujet à la lumière se trouvant dans le second état de mesure,
pour obtenir la première distribution de valeurs caractéristiques optiques sur la base du premier signal de détection et de la première distribution de valeurs d'intensité de la lumière, et
pour obtenir la seconde distribution de valeurs caractéristiques optiques sur la base du second signal de détection et de la seconde distribution de valeurs d'intensité de la lumière.

4. Dispositif d'obtention d'informations d'un sujet selon la revendication 3,
dans lequel le moyen de traitement du signal (250) est conçu pour obtenir une première distribution de pressions sonores initiales au sein du sujet (230) sur la base de la première pluralité de signaux de détection,
pour obtenir une seconde distribution de pressions sonores initiales au sein du sujet (230) sur la base de la seconde pluralité de signaux de détection,
pour obtenir la première distribution de valeurs caractéristiques optiques sur la base de la première distribution de pressions sonores initiales et de la première distribution de valeurs d'intensité de la lumière, et
pour obtenir la seconde distribution de valeurs caractéristiques optiques sur la base de la seconde distribution de pressions sonores initiales et de la seconde distribution de valeurs d'intensité de la lumière.

5. Dispositif d'obtention d'informations d'un sujet selon l'une quelconque des revendications 1 à 4,
dans lequel le moyen de traitement du signal (250) est conçu pour obtenir une première distribution de valeurs d'intensité de la lumière au sein du sujet (230) lors de l'exposition du sujet à la lumière se trouvant dans le premier état de mesure,
pour obtenir une seconde distribution de valeurs d'intensité de la lumière au sein du sujet (230) lors de l'exposition du sujet à la lumière se trouvant dans le second état de mesure,
pour obtenir une première région de confiance dans laquelle la valeur d'intensité de la lumière de la première distribution de valeurs d'intensité de la lumière est supérieure à un seuil,
pour obtenir une seconde région de confiance dans laquelle la valeur d'intensité de la lumière de la seconde distribution de valeurs d'intensité de la lumière est supérieure à un seuil, et
pour obtenir la distribution de valeurs de ressemblance sur la base de la première distribution de valeurs caractéristiques optiques, de la seconde distribution de valeurs caractéristiques optiques et de la première région de confiance, et de la seconde région de confiance.

6. Dispositif d'obtention d'informations d'un sujet selon l'une quelconque des revendications 1 à 4,
dans lequel le moyen de traitement du signal (250) est conçu pour obtenir une première distribution de valeurs d'intensité de la lumière au sein du sujet (230) lors de l'exposition du sujet à la lumière se trouvant dans le premier état de mesure,
pour obtenir une première région de confiance dans laquelle la valeur d'intensité de la lumière de la première distribution de valeurs d'intensité de la lumière est supérieure à un seuil, et
pour obtenir la distribution de valeurs de ressemblance sur la base de la première distribution de valeurs caractéristiques optiques, de la seconde distribution de valeurs caractéristiques optiques, et de la première région de confiance.

7. Dispositif d'obtention d'informations d'un sujet selon l'une quelconque des revendications 1 à 6,
dans lequel le moyen de traitement du signal (250) est conçu pour obtenir la distribution de valeurs de ressemblance sur la base d'une distribution de valeurs de corrélation entre la première distribution de valeurs caractéristiques optiques et la seconde distribution de valeurs caractéristiques optiques.

8. Dispositif d'obtention d'informations d'un sujet selon l'une quelconque des revendications 1 à 7, comprenant en outre un moyen d'affichage (280),
dans lequel le moyen de traitement du signal (250) est conçu pour afficher la distribution de valeurs de ressemblance sur le moyen d'affichage (280).

9. Dispositif d'obtention d'informations d'un sujet selon la revendication 8,
dans lequel le moyen de traitement du signal (250) est conçu pour obtenir une première distribution de valeurs d'intensité de la lumière au sein du sujet (230) lors de l'exposition du sujet à la lumière se trouvant dans le premier état de mesure,
pour obtenir une seconde distribution de valeurs d'intensité de la lumière au sein du sujet (230) lors de l'exposition du sujet à la lumière se trouvant dans le second état de mesure,
pour obtenir une première région de confiance dans laquelle la valeur d'intensité de la lumière de la première distribution de valeurs d'intensité de la lumière est supérieure à un seuil,
pour obtenir une seconde région de confiance dans laquelle la valeur d'intensité de la lumière de la seconde distribution de valeurs d'intensité de la lumière est supérieure à un seuil, et
pour afficher la distribution de valeurs de ressemblance et la première région de confiance et la seconde région de confiance sur le moyen d'affichage (280).

10. Dispositif d'obtention d'informations d'un sujet selon la revendication 8,
dans lequel le moyen de traitement du signal (250) est conçu pour obtenir une première distribution de valeurs d'intensité de la lumière au sein du sujet (230) lors de l'exposition du sujet à la lumière se trouvant dans le premier état de mesure,
pour obtenir une première région de confiance dans laquelle la valeur d'intensité de la lumière de la première distribution de valeurs d'intensité de la lumière est supérieure à un seuil, et
pour afficher la distribution de valeurs de ressemblance et la première région de confiance (280).

11. Dispositif d'obtention d'informations d'un sujet selon l'une quelconque des revendications 1 à 10,
dans lequel le détecteur d'ondes acoustiques comprend de multiples détecteurs d'ondes acoustiques à de multiples positions.

12. Dispositif d'obtention d'informations d'un sujet selon l'une quelconque des revendications 1 à 10,
comprenant en outre un moyen de balayage de détecteur d'ondes acoustiques (241) configuré pour balayer le détecteur d'ondes acoustiques.

13. Procédé d'obtention d'informations d'un sujet comprenant :

un processus (S30) pour obtenir une première distribution de valeurs caractéristiques optiques au sein d'un sujet (230) sur la base d'un premier signal de détection obtenu en détectant (S20) des ondes photoacoustiques générées en exposant le sujet à la lumière (S10) se trouvant dans un premier état de mesure ;
un processus (S60) pour obtenir une seconde distribution de valeurs caractéristiques optiques au sein du sujet (230) sur la base d'un second signal de détection obtenu en détectant (S50) des ondes photoacoustiques générées en exposant le sujet à la lumière (S40) se trouvant dans un second état de mesure ; et
un processus (S70) pour obtenir une distribution de valeurs de ressemblance entre la première distribution de valeurs caractéristiques optiques et la seconde distribution de valeurs caractéristiques optiques ;

dans lequel le premier état de mesure et le second état de mesure sont réglés (S10, S40) afin qu'ils soient différents en ce qui concerne la relation de position entre la lumière d'exposition, un détecteur d'ondes acoustiques (240) utilisé pour détecter les ondes photoacoustiques, et le sujet.

14. Programme qui, lorsqu'il est exécuté sur un ordinateur commandant le dispositif selon la revendication 1, amène le dispositif selon la revendication 1 à exécuter le procédé d'obtention d'informations d'un sujet selon la revendication 13.

FIG. 1A

FIG. 1B

FIG. 1C

FIG. 1D

FIG. 1E

SIMILARITY
HIGH

LOW

# FIG. 2

LIGHT SOURCE — 210

OPTICAL SYSTEM — 220

OPTICAL SCANNING MECHANISM — 221

SUBJECT — 230

ACOUSTIC WAVE DETECTOR SCANNING MECHANISM — 241

ACOUSTIC WAVE DETECTOR — 240

DISPLAY DEVICE — 280

SIGNAL PROCESSING DEVICE — 250

MEASUREMENT STATE SETTING MODULE — 251

OPTICAL CHARACTERISTIC VALUE DISTRIBUTION OBTAINING MODULE — 252

DATA PROCESSING MODULE — 253

LIGHT INTENSITY DISTRIBUTION OBTAINING MODULE — 254

CONFIDENCE REGION OBTAINING MODULE — 255

DATA OUTPUT MODULE — 256

MEMORY — 260

# FIG. 3

```
                    ┌──────────┐
                    │  START   │
                    └──────────┘
                         │
                         ▼
┌───────────────────────────────────────────────────┐
│           SET FIRST MEASUREMENT STATE               │───S10
└───────────────────────────────────────────────────┘
                         │
                         ▼
┌───────────────────────────────────────────────────┐
│            OBTAIN FIRST DETECTION SIGNAL            │───S20
└───────────────────────────────────────────────────┘
                         │
                         ▼
┌───────────────────────────────────────────────────┐
│  OBTAIN FIRST OPTICAL CHARACTERISTIC VALUE DISTRIBUTION │───S30
└───────────────────────────────────────────────────┘
                         │
                         ▼
┌───────────────────────────────────────────────────┐
│          SET SECOND MEASUREMENT STATE               │───S40
└───────────────────────────────────────────────────┘
                         │
                         ▼
┌───────────────────────────────────────────────────┐
│           OBTAIN SECOND DETECTION SIGNAL            │───S50
└───────────────────────────────────────────────────┘
                         │
                         ▼
┌───────────────────────────────────────────────────┐
│ OBTAIN SECOND OPTICAL CHARACTERISTIC VALUE DISTRIBUTION │───S60
└───────────────────────────────────────────────────┘
                         │
                         ▼
┌───────────────────────────────────────────────────┐
│     OBTAIN SIMILARITY DISTRIBUTION BETWEEN THE FIRST │
│  OPTICAL CHARACTERISTIC VALUE DISTRIBUTION AND SECOND │───S70
│      OPTICAL CHARACTERISTIC VALUE DISTRIBUTION       │
└───────────────────────────────────────────────────┘
                         │
                         ▼
┌───────────────────────────────────────────────────┐
│          DISPLAY THE SIMILARITY DISTRIBUTION        │───S80
└───────────────────────────────────────────────────┘
                         │
                         ▼
                    ┌──────────┐
                    │   END    │
                    └──────────┘
```

# FIG. 4A

445  430
425
420
440

# FIG. 4B

426  431  421
446
441

# FIG. 5A

545  530
525
520
540

# FIG. 5B

527  531  523
546
526
521
541

# FIG. 6A

# FIG. 6B

# FIG. 7A

# FIG. 7B

FIG. 8A

FIG. 8B

## FIG. 9A

995    991    996    992

## FIG. 9B

995    991    996    992

## FIG. 9C

993

# FIG. 10

SIMILARITY: 0.76

POSITION (X,Y,Z) = (121,342,40)

# FIG. 11

```
                    ┌──────────┐
                    │  START   │
                    └──────────┘
                          │
                          ▼
    ┌─────────────────────────────────────────────┐
    │        SET FIRST MEASUREMENT STATE           │──── S10
    └─────────────────────────────────────────────┘
                          │
                          ▼
    ┌─────────────────────────────────────────────┐
    │        OBTAIN FIRST DETECTION SIGNAL         │──── S20
    └─────────────────────────────────────────────┘
                          │
                          ▼
    ┌─────────────────────────────────────────────┐
    │     OBTAIN FIRST LIGHT INTENSITY DISTRIBUTION│──── S21
    └─────────────────────────────────────────────┘
                          │
                          ▼
    ┌─────────────────────────────────────────────┐
    │OBTAIN FIRST OPTICAL CHARACTERISTIC VALUE      │──── S30
    │DISTRIBUTION                                   │
    └─────────────────────────────────────────────┘
                          │
                          ▼
    ┌─────────────────────────────────────────────┐
    │        SET SECOND MEASUREMENT STATE          │──── S40
    └─────────────────────────────────────────────┘
                          │
                          ▼
    ┌─────────────────────────────────────────────┐
    │        OBTAIN SECOND DETECTION SIGNAL        │──── S50
    └─────────────────────────────────────────────┘
                          │
                          ▼
    ┌─────────────────────────────────────────────┐
    │    OBTAIN SECOND LIGHT INTENSITY DISTRIBUTION│──── S51
    └─────────────────────────────────────────────┘
                          │
                          ▼
    ┌─────────────────────────────────────────────┐
    │OBTAIN SECOND OPTICAL CHARACTERISTIC VALUE     │──── S60
    │DISTRIBUTION                                   │
    └─────────────────────────────────────────────┘
                          │
                          ▼
    ┌─────────────────────────────────────────────┐
    │OBTAIN SIMILARITY DISTRIBUTION BETWEEN THE     │
    │FIRST OPTICAL CHARACTERISTIC VALUE DISTRIBUTION│──── S70
    │AND SECOND OPTICAL CHARACTERISTIC VALUE        │
    │DISTRIBUTION                                   │
    └─────────────────────────────────────────────┘
                          │
                          ▼
    ┌─────────────────────────────────────────────┐
    │DISPLAY THE SIMILARITY DISTRIBUTION, FIRST     │
    │LIGHT INTENSITY DISTRIBUTION, AND SECOND       │──── S81
    │LIGHT INTENSITY DISTRIBUTION                   │
    └─────────────────────────────────────────────┘
                          │
                          ▼
                    ┌──────────┐
                    │   END    │
                    └──────────┘
```

# FIG. 12

SIMILARITY: 0.76

POSITION (X,Y,Z)=(121,342,40)

# FIG. 13

```
                    ( START )
                        │
                        ▼
┌──────────────────────────────────────────────┐
│         SET FIRST MEASUREMENT STATE            │──S10
└──────────────────────────────────────────────┘
                        │
                        ▼
┌──────────────────────────────────────────────┐
│         OBTAIN FIRST DETECTION SIGNAL          │──S20
└──────────────────────────────────────────────┘
                        │
                        ▼
┌──────────────────────────────────────────────┐
│      OBTAIN FIRST LIGHT INTENSITY DISTRIBUTION │──S21
└──────────────────────────────────────────────┘
                        │
                        ▼
┌──────────────────────────────────────────────┐
│         OBTAIN FIRST CONFIDENCE REGION         │──S22
└──────────────────────────────────────────────┘
                        │
                        ▼
┌──────────────────────────────────────────────┐
│  OBTAIN FIRST OPTICAL CHARACTERISTIC VALUE DISTRIBUTION │──S30
└──────────────────────────────────────────────┘
                        │
                        ▼
┌──────────────────────────────────────────────┐
│         SET SECOND MEASUREMENT STATE           │──S40
└──────────────────────────────────────────────┘
                        │
                        ▼
┌──────────────────────────────────────────────┐
│         OBTAIN SECOND DETECTION SIGNAL         │──S50
└──────────────────────────────────────────────┘
                        │
                        ▼
┌──────────────────────────────────────────────┐
│    OBTAIN SECOND LIGHT INTENSITY DISTRIBUTION  │──S51
└──────────────────────────────────────────────┘
                        │
                        ▼
┌──────────────────────────────────────────────┐
│        OBTAIN SECOND CONFIDENCE REGION         │──S52
└──────────────────────────────────────────────┘
                        │
                        ▼
┌──────────────────────────────────────────────┐
│ OBTAIN SECOND OPTICAL CHARACTERISTIC VALUE DISTRIBUTION │──S60
└──────────────────────────────────────────────┘
                        │
                        ▼
┌──────────────────────────────────────────────┐
│  OBTAIN SIMILARITY DISTRIBUTION BETWEEN THE FIRST │
│ OPTICAL CHARACTERISTIC VALUE DISTRIBUTION AND SECOND │──S70
│  OPTICAL CHARACTERISTIC VALUE DISTRIBUTION     │
└──────────────────────────────────────────────┘
                        │
                        ▼
┌──────────────────────────────────────────────┐
│ DISPLAY THE SIMILARITY DISTRIBUTION, FIRST CONFIDENCE REGION, │──S82
│      AND SECOND CONFIDENCE REGION              │
└──────────────────────────────────────────────┘
                        │
                        ▼
                    (  END  )
```

## FIG. 14A

1085      1081

1080

1497

1093

1496

SIMILARITY: 0.76

POSITION (X,Y,Z)=(121,342,40)

1086     1087

## FIG. 14B

1085      1081

1080

1497

1093

1496

SIMILARITY: 0.76

POSITION (X,Y,Z)=(121,342,40)

1086     1087

**EP 2 638 850 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010035806 A

- US 20100049049 A1

### Non-patent literature cited in the description

- **MINGHUA XU ; LIHONG V. WANG.** Universal back-projection algorithm for photoacoustic computed tomography. *PHYSICAL REVIEW,* 2005, vol. E 71, 016706